# EUROPEAN PATENT SPECIFICATION

(11) **EP 0 492 964 B1**
(45) Date of publication and mention of the grant of the patent: **08.08.2001**
(21) Application number: 91311758.6
(22) Date of filing: 18.12.1991
(51) Int. Cl.: C12N 15/31, C07K 14/20, C12N 15/62, C12N 1/21, C07H 21/04

(54) **Cloning and expression of Borrelia lipoproteins**
Klonierung und Expression von Borrelia Lipoproteinen
Clonage et expression de lipoprotéines de Borrelia

(30) Priority: 21.12.1990 US 632072
(43) Date of publication of application: 01.07.1992
(73) Proprietor: Brookhaven Science Associates LLC, Upton, New York 11973-5000 (US)
(72) Inventor: Dunn, John J., Bellport, NY 11713 (US); Barbour, Alan G., San Antonio, TX 78209 (US)
(74) Representative: Smart, Peter John

(56) References cited:
- PROTEIN EXPRESSION & PURIFICATION, vol. 1, no. 2, November 1990, pp. 159-168 JOHN J. DUNN ET AL. 'Outer surface protein A (OspA) from the Lyme disease spirochete, Borrelia burgdorferi: High level expression and purification of a soluble recombinant form of OspA'
- NUCLEIC ACIDS RESEARCH. vol. 17, no. 21, 11 November 1989, ARLINGTON, VIRGINIA US page 8864R. WALLICH ET AL. 'Cloning and sequencing of the gene encoding the outer surface protein A (OspA) of a European Borrelia burgdorferi isolate'
- Mol. Microbiol. (1989) 3: 479-486
- Infect. Immun. (1990) 58: 983-991

## Description

### BACKGROUND OF THE INVENTION

The present invention relates to a recombinant variation of the outer surface protein A (OspA) of Borrelia burgdorferi, the spirochete responsible for Lyme disease, and a method of producing the same.

Borrelia spirochetes are responsible for a variety of disorders such as Lyme disease. Lyme disease is an infection caused by the spirochete, Borrelia burgdorferi, which is carried by ticks. The spirochete is transmitted to humans and animals through the bite of a tick and can cause a serious dermatological, arthritic, neurological, and other pathological disorders in an infected host. Recently, Lyme disease has become a serious epidemiological concern in North America, as well as Europe, Asia and the Soviet Union.

It is well documented that persons and animals infected by Borrelia pathogens typically develop antibodies in response to the presence of various Borrelia antigens, including outer membrane lipoproteins. For example, patients infected with Lyme disease develop antibodies to outer surface protein A (OspA), a lioprotein of the Borrelia burgdorferi spirochete. See Craft, J.E., Fischer, D.K., Shimamoto, G.T., and Steere, A.C., "Antigens of Borrelia burgdorferi recognised during Lyme disease. Appearance of a new immunoglobulin in response and expansion of the immunoglobulin G response late in the illness, "J. Clin. Invest., 78: 934-939 (1986). See also, Barbour, A.G., Heiland, R.A, and Howe, T.R., "Heterogeneity of major proteins in Lyme disease borrelia: a molecular analysis of North American and European isolates,"J. Infect. Dis., 152: 474-484 (1985). The outer surface protein A (OspA) is a lipoprotein encoded by the nucleotide sequence of the ospA gene present in the DNA of the B. burgdorferi spirochete. The nucleotide sequence coding for the full-length, wild-type OspA (See SEQ ID NO: 1) has been previously determined for B31, the North American strain of B. burgdorferi. See Bergstrom, S., Bundoc, V.G., & Barbour, A.G., "Molecular Analysis of linear plasmid-encoded major surface proteins, OspA and OspB of the Lyme disease spirochete Borrelia burgdorferi, "Mol. Microbiol., 3: 479-486 (1989). Consequently, the OspA amino acid sequence has been predicted from the nucleotide data (see SEQ ID NO: 2).

From a clinical standpoint, it is highly desirable to develop a method of producing large quantities of highly purified Borrelia lipoproteins in a soluble form for use in immunoassays and other diagnostic screening tests which detect the presence of antibodies to these proteins in the sera of patients infected with Borrelia spirochetes. Furthermore, soluble, highly purified forms of these lipoproteins would be potentially valuable as a clinical immunogens for vaccinating both people and animals against Borrelia pathogens, as well as useful research tools for subsequent laboratory manipulations involving the separation and purification of antibodies to such proteins.

From the standpoint of recombinant DNA technology, it is highly desirable to obtain a nucleotide sequence or gene which can be expressed to very high levels (hyperexpression) in a recombinant host/vector expression system to yield large quantities of the resulting recombinant protein while retaining the desired specific reactivity.

Previous attempts have been made to isolate purified, soluble Borrelia lipoproteins through the growth and subsequent purification of Borrelia cell cultures. There are several drawbacks to this approach, however. The growth and subsequent purification of these proteins from crude cell extracts of Borrelia is very time consuming and expensive. Additionally, the growth and manipulation of live Borrelia cultures adds significant risk to laboratory personnel. Most importantly, the full-length, wild-type versions of Borrelia lipoproteins yielded by this method have poor solubility properties, as these proteins have a hydrophobic, lipidated character presumably due to their association with the cell membrane of the spirochete during expression. Consequently, detergents are required to solubilize these lipidated proteins.

It is well accepted in the art that the treatment of lipoproteins with detergents improves solubility but often impairs reactivity by altering or destroying the folding configuration of the target protein as well as the epitopic sites. Consequently, it would be desirable to develop a recombinant variation of OspA as well as other Borrelia lipoproteins that are soluble without exposure to detergents while retaining specific reactivity to antibodies against their full-length, wild-type lipoprotein analogs. In addition to the forgoing solubility problems, the association of the Borrelia lipoproteins with the outer cell membrane of the spirochete also creates problems in the separation and purification of these proteins from crude cell extracts.

Alternatively, certain recombinant DNA techniques can be utilized to express Borrelia genes using a host/vector expression system such as E. coli containing recombinant cloning vectors known in the art. A suitable recombinant cloning vector would be a plasmid having a nucleotide sequence that could be modified to accept an insertion of wild-type Borrelia DNA. While these recombinant techniques avoid the need for live Borrelia cultures, they have several shortcomings.

For example, recombinant versions of the full-length, wild-type Borrelia burgdorferi produced using E.coil have poor solubility properties in the absence of detergents, presumably due to the association of the protein with the outer cell membrane of the host during expression. Consequently, subsequent manipulations directed to the separation and purification of the resulting protein product involve problems similar to those encountered when attempting to isolate and purify OspA from live B, burgdorferi cultures.

Another shortcoming of the above approach is that recombinant versions of the full-length, wild-type OspA gene undergo poor hyperexpression in an E.coli host. This poor expression is presumably due to the accumulated toxic effects of OspA protein localisation at the E.coli cell membrane during the course of expression.

The present invention, in one aspect, is directed towards the provision of a non-lipidated protein comprising an amino acid sequence which consists of the sequence SEQ ID NO:10 with one or more alanine and/or methionine residues at the N-terminal end of the SEQ ID NO:10 sequence.

The present invention thus provides a recombinant variation of B. burgdorferi outer surface protein A (OspA) which is soluble without exposure to detergents while retaining specific reactivity to antibodies against wild-type B. burgdorferi OspA.

The modified OspA is not associated with the host cell membrane during expression.

The present invention, in addition, is directed towards a method for providing a truncated version of a nucleotide sequence coding for the Borrelia burgdorferi lipoprotein OspA, comprising:
providing a DNA template comprising said nucleotide sequence coding for said Borrelia lipoprotein, said template containing a potential signal peptidase II signal sequence, wherein said signal sequence has a 5' end and a 3' end, said 3' end terminating in a codon for a cysteine residue; and
synthesizing a set of oligonucleotide primers comprising a first and a second primer, said first primer comprising a region which is sufficiently complementary to a first DNA strand of said DNA template for effectively priming amplification of said first DNA strand in a 5' to 3' direction and a second primer comprising a region which is sufficiently complementary to a second DNA strand of said DNA template for effectively priming amplification of said second DNA strand in a 5' to 3' direction, and using said primers is an amplification reaction wherein said first primer initiates a DNA polymerisation at a nucleotide positioned on said DNA template downstream from said cysteine residue in a 3' direction. Thus, there is provided a nucleotide sequence that is highly expressed in a host organism such as E.coli and which codes for a recombinant variation of B. burgdorferi OspA which is soluble without exposure to detergents while retaining specific reactivity to antibodies against wild-type B. burgdorferi OspA.

The method for providing the protein of the present invention involved producing a truncated version of the wild-type B. burgdorferi OspA gene which could be highly expressed in a recombinant host to yield a soluble product. Using a DNA template containing B. burgdorferi DNA, specially designed oligonucleotide primers were utilised in a polymerase chain reaction to amplify a segment of the wild-type OspA gene which excluded the first 17 codons that contain a signal peptidase II signal sequence. The resulting amplification product was expressed in a T7 bacteriophage expression system using recombinant DNA techniques known in the art.

A DNA plasmid, pET9-OspA, harboring the nucleotide sequence coding for the protein of the present invention is also provided, in addition to a strain of E.coli transformed by the same.

The protein of the present invention is highly advantageous in that it retains reactivity to antibodies against wild-type B. burgdorferi OspA while maintaining improved solubility properties over OspA derived from live cultures or other recombinant techniques. This improved solubility is particularly advantageous in immunodiagnostic assays as well as laboratory manipulations because the protein is soluble in the absence of detergents which can impair or destroy reactivity.

Another advantage of the protein of the present invention is that it is not associated with the host cell membrane recombinantly. Consequently, the protein of the present invention can be expressed to high levels using recombinant techniques because it is not as toxic to the host organism as wild-type OspA. Improved recombinant expression affords high yields of the target protein while obviating the risks and expenses of live Borrelia cell cultures.

A further advantage of the present invention is that it provides a method of producing a recombinant variation of highly expressed Borrelia lipoproteins having improved solubility in the absence of detergents while retaining specific reactivity to antibodies against their wild-type, lipoprotein analogs. Prior to the method of the present invention, detergents were required to solubilise these lipoproteins for use in immunoassays and other laboratory manipulations, thereby exposing reactive epitopic sites to the potentially damaging effects of the detergents.

Accordingly, in one broad aspect of the present invention, there is provided a protein encoded by a nucleic acid sequence comprising a first and a second portion, wherein the second portion has a 5' and a 3' end and comprises SEQ ID NO: 10 and wherein the first portion is positioned at the 5' end of the second portion and consists of a nucleotide sequence coding for an amino acid sequence at least one of alanine and methionine. The amino acid sequence may comprise SEQ ID NO:4, SEQ ID NO:6 or SEQ ID NO:8, as set forth below and the nucleotide sequence may comprise SEQ ID NO:3, SEQ ID NO:5 or SEQ ID NO:7, as set forth below.

In another broad aspect of the invention, there is provided a protein encoded by an amino acid sequence SEQ ID NO:10, as set forth below, as well as a nucleotide sequence coding for the amino acid sequence, which may comprise SEQ ID NO:9.

Further specific aspects of the invention include a pET9 plasmid for transforming a host organism comprising pET-OspA; a transformed strain of E.coli containing plasmid pET9-OspA, wherein the strain is BL21(DE3)/pLysS, pET9-OspA; and an oligonucleotide primer useful for an amplification of a portion of a nucleotide sequence coding for a Borrelia lipoprotein wherein the primer is SEQ ID NO:11.

Another aspect of the invention provides a truncated version of a nucleotide sequence coding for the Borrelia burgdorferi lipoprotein OspA, comprising: (a) providing a DNA template comprising the nucleotide sequence coding for the Borrelia lipoprotein, the template containing a potential signal peptidase II signal sequence, wherein the signal sequence has a 5' end and a 3' end, and 3' end terminating in a codon for a cysteine residue; and (b) synthesizing a set of oligonucleotide primers comprising a first and a second primer, the first primer comprising a region which is sufficiently complementary to a first DNA strand of the DNA template for effectively priming amplification of the first DNA strand in a 5' to 3' direction and a second primer comprising a region which is sufficiently complementary to a second DNA strand of the DNA template for effectively priming amplification of the second DNA strand in a 5' to 3' direction, wherein the first primer initiates a DNA polymerisation at a nucleotide positioned on the DNA template downstream from the cysteine residue in a 3' direction.

An additional aspect of the invention provides a method for providing a recombinant variation of the Borrelia burgdorferi lipoprotein OspA comprising: (a) providing a DNA template comprising a nucleotide sequence coding for said Borrelia lipoprotein, the template containing a potential signal peptidase II signal sequence, wherein the signal sequence has a 5' end and a 3' end, the 3' end terminating in a codon coding for a cysteine residue; (b) synthesizing a set of oligonucleotide primers comprising a first and a second primer, the first primer comprising a region which is sufficiently complementary to a first DNA strand of the DNA template for effectively priming amplification of the first DNA strand in a 5' to 3' direction and a second primer comprising a region which is sufficiently complementary to a second DNA strand of the DNA template for effectively priming amplification of the second DNA strand in a 5' to 3' direction, wherein the first primer initiates a DNA polymerization at a nucleotide positioned on said DNA template downstream from the cysteine residue in a 3' direction; (c) reacting the primers in a polymerase chain reaction thereby providing a set of amplification products; (d) isolating a truncated version of the nucleotide sequence from the set of amplification products; (e) cloning the truncated version into a suitable expression vector; (f) transforming a suitable host organism using the expression vector; and (g) cultivating the host organism for production of the recombinant variation of the lipoprotein.

For a better understanding of the present invention together with other and further objects, reference is made to the following description, taken together with the accompanying drawings, and its scope will be pointed out in the appended claims.

### BRIEF DESCRIPTION OF THE DRAWINGS

Fig. 1 depicts the nucleotide sequence of oligonucleotide primer 201->216 schematically.

Fig. 2 depicts the nucleotide sequence of oligonucleotide primer 958<-972 schematically.

Fig. 3 depicts the nucleotide sequence of oligonucleotide primer 151->171 schematically.

Fig. 4 depicts the wild-type ospA gene schematically, highlighting the positions where primers 201->216 and 958<-972 anneal.

Fig. 5 depicts the wild-type ospA gene schematically, highlighting the positions where primers 151->171 and 958<-972 anneal.

Fig. 6 depicts the product resulting from the amplification of the wild-type ospA gene by primer 201->216 and primer 958<-972.

Fig. 7 is a photograph of a one percent agarose gel stained with ethidium bromide upon which amplification products were run.

Fig. 8 is a schematic representation of plasmid pET9.

Fig. 9 is a schematic representation of plasmid pET9-OspA.

Fig. 10 is a photograph of a SDS-12.5% PAGE gel stained with Coomassie blue upon which various cellular protein samples were run subsequent to removal from induction at specified time intervals.

Fig. 11 is a photograph of a SDS-PAGE gel upon which uninduced cells were compared with induced cells sampled at one hour intervals after induction.

Fig. 12 is a photograph of a SDS-PAGE gel upon which OspA samples were run. The samples were taken at different stages of purification.

Fig. 13 is an autoradiogram of a Western blot immunochemical analysis of OspA and OspB proteins from whole B. burgdorferi cells as well as OspA and preOspA.

Fig. 14 is a photograph of a SDS-12.5% PAGE gel stained with Coomassie blue upon which cells carrying pET9-preOspA and cells carrying pET9-OspA were compared to whole B. burgdorferi cells.

Fig. 15 is an autoradiogram of the gel photographed in Fig. 14.

Fig. 16 is an autoradiogram of the nitrocellulose blot of the gel photographed in Fig. 14 before further Western analysis.

Fig. 17 is an autoradiogram of the nitrocellulose blot of the gel photographed in Fig. 14 after probing with antibodies.

Fig. 18 is a photograph of the completed Western blot of the gel photographed in Fig. 14 after treatment with alkaline phosphatase color developing reagents.

### DETAILED DESCRIPTION OF THE INVENTION

For purposes of understanding the present invention, the following terms are defined:

"Bacteria" are prokaryotic organisms that possess a tough protective coat known as a cell wall beneath which a cell membrane encloses a single cytoplasmic compartment containing DNA, RNA, proteins and small molecules. Examples include spirochetes and Escherichia coli.

"Hyperexpression" is a high level expression of cloned genes. Bacteriophage T7 RNA polymerase can direct high level transcription from a T7 promoter on a multicopy plasmid, efficiently transcribing almost any DNA linked to a T7 promoter. This results in high level expression of the linked DNA.

A "plasmid" is a double-stranded, closed, circular DNA molecule independent of the chromosome and comprising an intact replicon such that the plasmid is replicated in a host cell. when the plasmid is placed within a cell of a unicellular organism, the characteristics of the organism may be transformed. Among the phenotypes conferred by plasmids are resistance to antibiotics and production of restriction and modification enzymes.

"Primer" refers to an oligonucleotide (a short nucleic acid chain), which is capable of acting as a point of initiation of synthesis when placed under conditions in which synthesis of a primer extension product, which is complementary to a nucleic acid strand, is induced. The primer may occur naturally, as in a purified restriction digest or be produced synthetically.

It has now been found that redesigning the wild-type ospA gene (see SEQ ID NO: 1) to remove the first 17 codons from the nucleotide sequence yields a truncated nucleotide sequence which can be hyperexpressed in a host organism such as E. coli to produce a recombinant variation of B. burgdorferi OspA which is soluble without exposure to detergents but retains a selective reactivity to antibodies against wild-type B. burgdorferi OspA. It is the presence of the first 17 codons which has accounted for the shortcomings in prior art attempts to reproduce a soluble recombinant form of OspA that is highly expressed in E. coli.

More precisely, a region of nucleotides (see SEQ ID NO: 14) appears within the leader sequence (the first 17 codons in the wild-type, B. burgdorferi ospA gene) coding for a signal which triggers lipidation of the resulting protein, thereby impairing both solubility as well as expression during processing within a recombinant host such as E. coli.

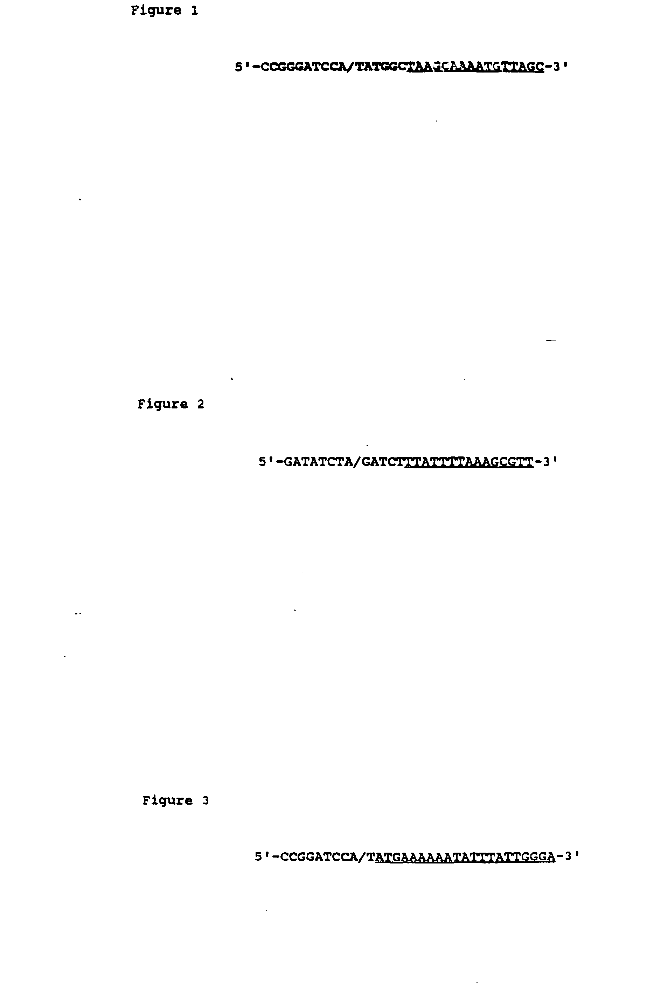

DNA containing the full-length, wild-type B. burgdorferi (B31 strain) ospA gene was isolated and purified. A set of oligonucleotide primers (see SEQ ID NO: 11/Fig. 1 and SEQ ID NO: 12/Fig. 2) was synthesized for use in the polymerase chain reaction allowing for the specified amplification of a truncated version of the ospA gene lacking the first 17 codons as well as providing restriction sites before and after the coding sequence of the amplified product. A second set of oligonucleotide primers (see SEQ ID NO: 13/Fig. 3 and SEQ ID NO: 12/Fig. 2) was also synthesized for use in the polymerase chain reaction to allow for the specified amplification of the entire wild-type ospA gene as a control mechanism.

The resulting nucleotide fragments produced by the polymerase chain reaction were purified and selected by restriction site analysis and subsequently subcloned into an appropriate plasmid expression vector that was compatible from the standpoint of restriction sites. The resulting plasmids were then transferred to a host expression strain for protein production.

### EXAMPLE 1

In order to construct the protein of the present invention, it was necessary to procure a source of wild-type B. burgdorferi DNA containing the nucleotide sequence coding for OspA. This DNA served as a template for the amplification of the desired segment of the wild-type, ospA gene (see SEQ ID NO: 1) during the polymerase chain reaction. It is well known in art that starting material for recombinant DNA manipulations can be DNA isolated from cultures of the wild-type organism of interest or recombinant vehicles, such as plasmids, that have been genetically engineered to contain cloned copies of the target DNA. The latter approach is advantageous in that it promotes homogeneity and reduces the frequency of mutation in the DNA fragment of interest.

In the preferred method of producing the protein of the present invention, the initial source of template DNA containing the full-length, wild-type ospA gene was a recombinant clone of the ospA gene obtained from a previously engineered plasmid, pTRH44. The plasmid pTRH44, having a 1.6-kb restriction fragment containing the full-length, wild type B. burgdorferi ospA gene cloned into pUC9, has been previously described. See Howe, T.R., LaQuier, F.R., and Barbour, A.G., "Organization of genes encoding two outer membrane proteins of the Lyme disease agent Borrelia burgdorferi within a single transcriptional unit," Infec. Immun., 54: 207-212 (1986).

Alternatively, total Borrelia Burgdorferi DNA could have been isolated and purified by phenol extraction or lysozyme-proteinase K-SDS extract of cells harvested from stationary phase cultures for use as template DNA. Techniques for isolation and purification of template DNA are generally well known in the art. For example, see Howe, T.R., Mayer, L.W., & Barbour, A.G., "A single recombinant plasmid expressing two major outer surface proteins of the Lyme disease spirochete," Science 227: 645-646, (1985). For examples on cultivation and isolation of B. burgdorferi see Barbour, A.G., "Isolation and cultivation of Lyme disease spirochetes," Yale J. Biol. Med., 57: 521-525 (1984).

A first and second set of oligonucleotide primers were synthesized in a Microsyn 1450 DNA synthesizer (available from Systec, Minneapolis, Minnesota). The resulting products were subsequently purified using Poly-Pak^{R} purification cartridges (obtained from Glen Research Corporation, Herndon, Virginia) according to the manufacturer's specifications. DNA synthesis and subsequent purification techniques are well known in the art of recombinant DNA technology. Any suitable techniques for achieving these steps would be acceptable.

The first set of oligonucleotide primers was designed for the amplification of a nucleotide sequence coding for a recombinant variation of B. burgdorferi OspA while the second set of primers was designed for the amplification of the entire wild-type B. burgdorferi ospA gene. Each primer contained a 5' end and a 3' end. The 3' end of each primer contained a region having a nucleotide sequence complementary to a specific sequence of nucleotides appearing at a particular segment of the wild-type B. burgdorferi ospA gene present within the B. burgdorferi genome. It was this region of the primer that annealed to the B. burgdorferi DNA template to promote polymerization during the polymerase chain reaction. The nucleotide sequence for the wild-type ospA gene (see SEQ ID NO: 1) has been previously determined. See Bergstrom, S., Bundoc, V.G., & Barbour, A.G., "Molecular Analysis of linear plasmid-encoded major surface proteins, OspA and OspB, of the Lyme disease spirochete Borrelia burgdorferi," Mol. Microbiol., 3: 479-486 (1989).

The 5' end of each primer contained a nucleotide sequence that was non-complementary to the B. burgdorferi DNA template while introducing restriction sites in the fragments produced during amplification. It was the presence of the restriction sites which facilitated the cloning of the resulting fragments into an expression vector. The use of restriction sites to facilitate cloning is well known in recombinant DNA technology.

The first set of oligonucleotide primers included a first and a second primer. The first primer was denoted as primer 201->216 and was synthesized to yield the nucleotide sequence (SEQ ID NO: 11) shown in Fig. 1. The numbers 201-216 indicate the specific nucleotide positions on the full-length, wild-type ospA gene to which the primer was complementary. Referring to Fig. 1, the underlined region indicates the segment of the primer which was complementary to the full-length, wild-type ospA gene at nucleotide positions 201 through 216. The nucleotides appearing in boldface print indicate a restriction site recognized by restriction enzyme NdeI. The slash mark represents the site where the NdeI enzyme later cleaved the strand to facilitate cloning into the expression vector.

Primer 201->216 was used to redesign a 5' end for the truncated ospA gene (the nucleotide sequence coding for the recombinant variation of OspA) providing a NdeI restriction site and priming the amplification from the full-length, wild-type ospA gene by initiating polymerization at the 18th codon. In the wild-type version of the ospA gene, a potential recognition site for lipoprotein signal peptidase II occurs between the 16th and 17th codon.

The exact mechanism for lipidation of the fulllength, wild-type OspA within the Borrelia spirochete is not known. However, it is now generally accepted in the art that the amino acid sequence Leu-x-y-Cys (where x and y generally are different amino acids having nonpolar side chains), appearing in the leader sequence of certain bacterial lipoproteins, codes for a processing signal to initiate protein processing by the bacterial enzyme, signal peptidase II. This enzyme is ultimately responsible for cleaving the N-terminal portion of the leader sequence at the amino end of the cysteine residue, leaving the N-terminal cysteine to be covalently linked to fatty acids which give the remaining protein a highly lipidated character upon attachment. Many prokaryotic cells such as E. coli utilize the foregoing processing scheme to process and transfer their own cellular lipoproteins to the membrane of the cell. See Bergstrom, S., Bundoc, V.G., and Barbour, A.G., "Molecular Analysis of linear plasmid-encoded major surface proteins, OspA and OspB, of the Lyme disease spirochete Borrelia burgdorferi," Mol. Microbiol., 3: 479-486 (1989). See also Brandt, M.E., Riley, B.S., Radolf, J.D., and Norgard, M.V., "Immunogenic integral membrane proteins of Borrelia burgdorferi are lipoproteins," Infect. Immun., 58:983-991 (1990).

Although the entire amino acid sequence of the wild-type version of B. burgdorferi OspA has not been confirmed by amino acid analysis due to problems inherent in the protein, the sequence has previously been predicted based upon the known nucleotide sequence of the full-length, wild-type B. burgdorferi (B31) ospA gene. See Bergstrom, S., Bundoc, V.G., and Barbour, A.G., "Molecular Analysis of linear plasmid-encoded major surface proteins, OspA and OspB, of the Lyme disease spirochete Borrelia burgdorferi," Mol. Microbiol., 3: 479-486 (1989). SEQ ID NO: 2 illustrates the predicted amino acid of sequence of the full-length, wild-type B. burgdorferi OspA as previously deduced.

Referring to SEQ ID NO: 2, it can be seen that the leader portion or the predicted amino acid sequence contains a segment having the consecutive amino acid residues Leu-Ile-Ala-Cys (see SEQ ID NO: 15). These residues conform to the format of the processing signal for signal peptidase II in E. coli, as mentioned above. Thus in the wild-type version of the ospA gene, a potential recognition site for lipoprotein signal peptidase II occurs between the 16th and 17th codon due to the sequence homology between the known signal sequence format for signal peptidase II and the potential signal sequence appearing in the predicted amino acid sequence of the full-length, wild-type B. burgdorferi OspA.

To increase the likelihood that the resulting recombinant protein would not become lipidated during hyperexpression, the complementary segment of primer 201->216 was designed to exclude the cysteine residue and to initiate amplification at the portion of the B. burgdorferi wild-type ospA gene beginning at the 18^{th} codon in order to completely eliminate the potential recognition site for lipidation. It was hoped that the elimination of this potential recognition site would increase solubility and improve expression of the resulting protein without impairing specific reactivity to antibodies against wild-type B. Burgdorferi OspA.

Prior to the present invention, it was unknown as to whether the potential signal sequence appearing in the amino acid sequence of wild-type B. burgdorferi OspA was responsible for the lipidation of the mature protein. It was further unknown as to whether the potential signal sequence would be involved in a similar lipidation or a recombinant version of wild-type OspA produced in a recombinant host. It was yet further unknown as to whether the elimination of a portion of the wild-type ospA gene containing the potential signal sequence would result in a truncated ospA gene which could be effectively expressed using recombinant methods to yield a protein having improved solubility in the absence of detergents while retaining reactivity to antibodies against the wild-type version of the protein.

It is well accepted in the art of recombinant DNA technology that the greater the degree of modification imposed upon a naturally-occuring nucleotide sequence coding for a protein, the greater the risk becomes that the resulting recombinant protein will suffer from altered reactivity, if it is even expressed at all. Indeed, such a recombinant transfer may prove toxic or fatal to the host organism thereby reducing or eliminating expression of the desired product.

Referring to Fig. 1, the preferred construction of primer 201->216 called for a non-complementary segment (GCT), coding for an alanine residue, to be positioned between the complementary segment of the primer and the NdeI restriction site. The NdeI restriction site positioned within primer 201->216 included a triplicate (ATG) coding for methionine, a terminal amino acid residue which functions as an initiation site during protein production. The triplicate coding for alanine was added because alanine is one of the amino acids which facilitates the removal of methionine from the final protein product. Other amino acids suitable for facilitating the removal of methionine would also be acceptable. See Hirel, P-H., Schmitter, J-M., Dessen, P., Fayat, G., & Blanquet, S., "Extent of N-terminal methionine excision from Escherichia coli proteins is governed by the side-chain length of the penultimate amino acid," Proc. Natl. Acad. Sci. U.S.A., 86: 8247-8251 (1989). It is generally known that methionine appearing at the terminal end or an amino acid sequence for the purpose of translation initiation is not important to the characteristics of the resulting protein and is usually removed from the amine acid sequence.

In the preferred form of the present invention, the amino acid sequence resulting from the truncated ospA gene had an additional alanine residue at the amino terminal end. Since the first 17 codons were eliminated from the truncated version of the ospA gene, the nucleotide triplicate coding for the alanine residue was positioned to precede the nucleotide triplicate coding for a lysine residue at what would be the 18^{th} codon in the wild-type ospA gene. Methionine was removed in the mature form of the expressed protein. The resulting amino acid sequence with methionine removed is illustrated in SEQ ID NO: 6. The corresponding nucleotide sequence is shown in SEQ ID NO: 5.

Alternatively, the additional alanine residue could have been left out. The resulting amino acid sequence including the initiating methionine is illustrated in SEQ ID NO: 8. The corresponding nucleotide sequence is shown in SEQ ID NO: 7. Removal of methionine results in amino acid sequence SEQ ID NO: 10. The corresponding nucleotide sequence is shown in SEQ ID NO: 9.

The second primer was denoted as primer 958<-972 and was synthesized to yield the sequence (see SEQ ID NO: 12) shown in Fig. 2. Referring to Fig. 2, the underlined region of the primer indicates the segment which was complementary to the wild-type ospA gene at positions 958 through 972 while the nucleotides appearing in boldface print indicate a restriction site recognized by restriction enzyme BglII. The slash mark represents the site where the BglII enzyme later cleaved the product strand to facilitate cloning into the expression vector. Referring to SEQ ID NO: 12, it can be seen that the entire sequence is shown in a noncoding format as contrasted with the format presented in SEQ ID NOS: 11 and 13, which correspond to primer 201->216 and primer 151->171, respectively. The noncoding format was used because the sequence of primer 958<-972 is actually designed to prime the amplification of the non-sense strand of the ospA gene rather than the sense strand.

Primer 958<-972 was common to both sets of oligonucleotide primers. Insofar as the first set of primers was concerned, primer 958<-972 was used to redesign a 3' end for the truncated ospA gene providing a BglII restriction site and priming the amplification in a direction antiparallel to the direction of amplification directed by primer 201->216.

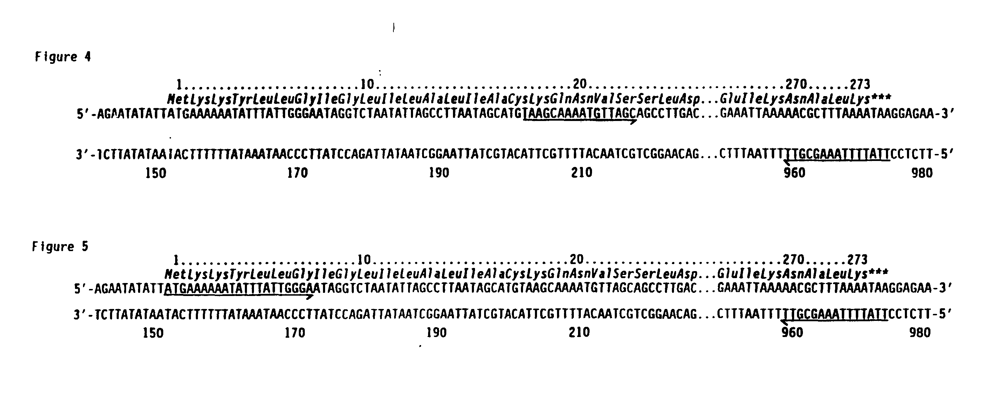

Referring to Fig. 4, the wild-type ospA gene is depicted schematically. The two regions of nucleotides underlined highlight the positions where primer 201->216 and primer 958<-972 annealed to the template DNA to promote amplification. The arrowheads denote the direction of polymerization initiated by the primer which annealed at the position indicated.

The second set of oligonucleotide primers, designed for the amplification of the entire wild-type B. burgdorferi ospA gene as a control, also included a first and a second primer. The first primer was denoted as primer 151->171 and was synthesized to yield the nucleotide sequence (see SEQ ID NO: 13) shown in Fig. 3. Referring to Fig. 3, the underlined region indicates the segment of the primer which was complementary to the wild-type ospA gene at nucleotide positions 151 through 171. The nucleotides appearing in boldface print indicate a restriction site recognized by restriction enzyme NdeI. The slash mark indicates the site where the NdeI enzyme later cleaved the product strand to facilitate cloning into the expression vector.

The second primer, primer 958<-972, was common to both sets of oligonucleotides as previously mentioned. Insofar as the second set of primers was concerned, primer 958<-972 introduced a BglII restriction site and primed the amplification of the wild-type ospA gene in a direction antiparallel to the direction of amplification directed by primer 151->171.

Referring to Fig. 5, the wild-type ospA gene is depicted schematically. The two regions of nucleotides underlined highlight the positions where primer 151->171 and primer 958<-972 annealed to promote amplification. The arrowheads denote the direction of polymerization initiated by the primer which annealed at the position indicated.

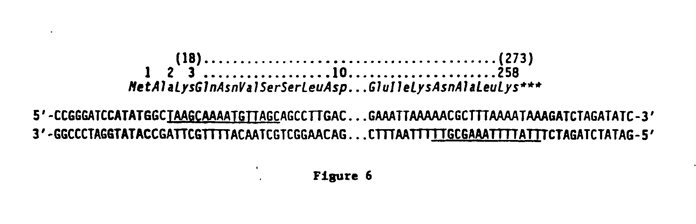

Fig. 6 is a schematic illustration of the product resulting from the amplification of the truncated version of the ospA gene from the wild-type ospA gene by primer 201->216 and primer 958<-972. The boldface print denotes the restriction sites provided by the primers while the underlined regions indicate the section of the primer which annealed to the wild-type ospA gene prior to amplification.

The basic methods for amplifying a desired target nucleic acid sequence using oligonucleotide primers are generally known in the art and are illustrated in U.S. Patent No. 4,683,202 to Mullis and U.S. Patent No. 4,800,159 to Mullis, et al., both of which are incorporated herein by reference. For additional information concerning cloning techniques, see Maniatis, T., Fritsch, E.F., & Sambrook, J., Molecular Cloning: A Laboratory Manual, Cold Spring Harbor Laboratory, Cold Spring Harbor, N.Y. (1982). See also, Ausubel, F.M., Brent, R., Kingston, R.E., Moore, D.D., Seidman, J.G., Smith, J.A., & Struhl, K., Current Protocols in Molecular Biology, John Wiley & Sons, New York, N.Y. (1989).

Utilizing primers 151->171, 201->216 and 958<-972, polymerase reaction amplifications were carried out in 50 *µ*l reaction volumes containing 1 unit AmpliTaq DNA polymerase (obtained from Perkin-Elmer Cetus, Norwalk, CT), each primer at 1 *µ*M an ~ 0.1 *µ*g template DNA. The reaction mix also contained 10 mM Tris-HCl (pH 8.0), 50 mM KCl, 1.5 mM MgCl₂, 0.05% Tween 20, 0.05% Nonedet P-40, 0.26 mM dATP, 0.26 mM dTTP, 0.14 mM dCTP and 0.14 mM dGTP.

In the preferred method of amplification, the dNTP concentration used reflected the known DNA base content of 29% G + C for B. burgdorferi to eliminate the possibility or undesirable mutations. See Schmid, G.P., Steigerwalt, A.G., Johnson, S., Barboug, A.G., Steere, A.C., Robinson, I.M. and Brenner, D.J., "DNA characterization of Lyme disease spirochetes," Yale J. Biol. Med., 57:539-542 (1984).

Before amplification, the reaction was overlaid with mineral oil and amplification was carried out for 25 cycles in a DNA Thermal Cycler (obtained from Perkin-Elmer Cetus, Minneapolis, MN.) with each cycle consisting of 1 min at 94°C, 1 min at 47°C and 3 min at 72 °C. Amplification was completed by a final incubation at 72°C for 10 min. The amplified products were extracted with phenol, ethanol precipitated, cleaved with the appropriate restriction enzymes and then purified by electrophoresis on 1% low melting point agarose gels (obtained from Bethesda Research Laboratories, Gaithersburg, MD). other techniques known in the art for purification of amplified DNA products, such as electrophoresis on acrylamide gels, would be acceptable. It is well known in the art that the amplified products must be purified and treated with the appropriate restriction enzymes (NdeI and BglII for present example) before expression can be initiated.

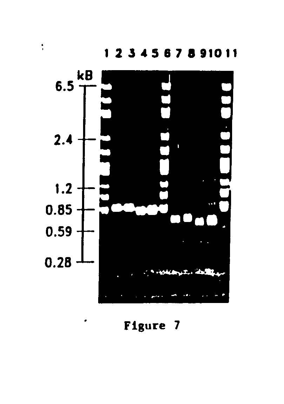

The amplification products were run on a 1% agarose gel, stained with ethidium bromide and photographed with UV illumination. Referring to Fig. 7, Lanes 1, 6 and 11 contain HaeII digested T7 DNA as molecular length markers. Sizes are in kilobase pairs (kB). Lanes 2, 4, 7 and 9 contain 1/5 vol of the products of the reactions with total B. burgdorferi DNA while lanes 3, 5, 8 and 10 contain 1/50 volume of the reaction mix resulting from amplification using plasmid pTRH44 as a template. The samples applied were generated using primers that amplify the entire ospA coding sequence (Lanes 2, 3, 7 and 8) or the region beginning at Lys¹⁸ (Lanes 4, 5, 9 and 10). As shown in Lanes 7-10, the amplified DNA can be cut with EcoRI to give products with the mobilities expected from cutting at the single EcoRI site in ospA (662 + 182, and 623 + 182 bps, respectively).

### EXAMPLE 2

In order to express the amplified version of the truncated ospA gene as well as the amplified version of the full-length, wild-type ospA gene, the DNA fragments resulting from amplification by the polymerase chain reaction were ultimately cloned into a plasmid vector for protein production. The preferred plasmid vector for protein production in the present invention was pET9 in which the ospA gene is placed under control of a T7 promoter and efficient translation initiation signals from bacteriophage T7. The pET9 and pLysS expression vectors, the bacterial hosts for cloning, growth media and the methods used to direct expression of cloned genes by T7 RNA polymerase have previously been described. See Studier, F.W., Rosenberg, A.H., Dunn, J.J. & Dubendorff, J.W., Meth. Enzymol., "Use of T7 RNA polymerase to direct expression of cloned genes," 185: 60-89 (1990). Cloning and expression of the gene for bacteriophage T7 RNA polymerase is also presented in U.S. Patent No. 4,952,496 to Studier, et al., incorporated herein by reference. While a T7 promoter system is the preferred expression system in the present invention, expression of the truncated ospA gene is not to be so limited with respect to expression format, provided that the expression system of choice is compatible with the host organism.

The resulting plasmids were designated as pET9-preOspA, denoting the plasmids which received the amplified DNA fragment coding for the full-length, wild-type OspA used as a control, and pET9-OspA, denoting the plasmids which received the amplified DNA product coding for the recombinant variation of OspA.

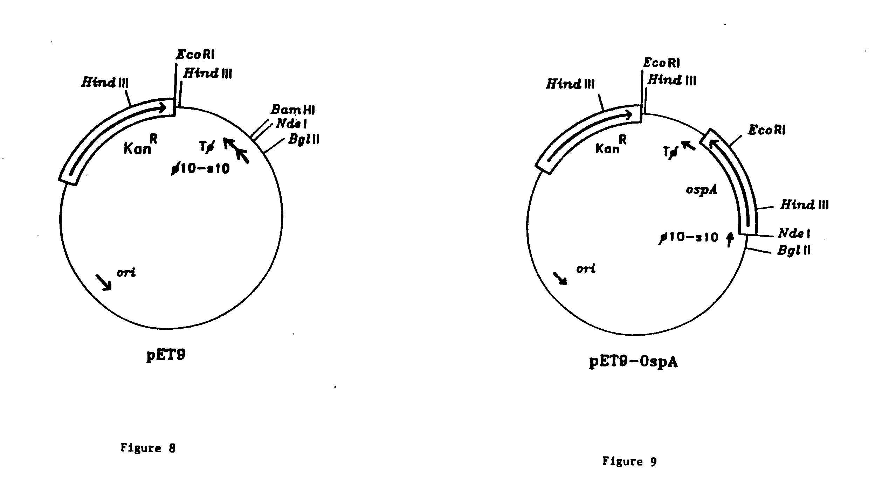

In the preferred form of the present invention, the pET9 vector was used since it has a kan gene as its selective marker instead of a bla gene. Consequently, ampicillin is not used during cell growth and, therefore, there is no possibility that an immunogenic ampicilloyl/OspA target protein conjugate can be formed. Conjugates of this type are believed to be major antigenic determinants in penicillin allergy and their presence could complicate projected immunological studies. See Yvon. M., Anglade, P., & Wal, J-M., "Identification of the binding sites of benzyl penicilloyl, the allergenic metabolite of penicillin, on the serum albumin molecule," FEBS, 263: 237-240 (1990). A schematic representation of pET9 and pET9-OspA plasmids are shown in Figs. 8 and 9, respectively.

Plasmids pET9-preOspA and pET9-OspA were initially cloned into DH5α, a host that lacks T7 RNA polymerase. Background expression is minimal in this host since the bacterial RNA polymerase does not initiate from the T7 promoter. In principle, stable recombinant plasmids can be established in this host even though the target gene product is toxic to E. coli. The correctness of the resulting plasmids was confirmed by extensive restriction site analysis and standard dideoxy sequencing of the entire ospA coding sequences.

Although the primers used here were specifically designed to amplify certain segments of the wild-type ospA sequence from total B. burgdorferi DNA, for practical reasons and because the mutation probability increases with the number of amplification cycles, the plasmids used in the present invention were constructed using NdeI/BglII fragments obtained from reactions containing the ospA plasmid, pTRH44, as a DNA template. Subsequently, the resulting 824 and 779 bp NdeI/BglII fragments from each reaction were subcloned separately into the T7 expression vector pET9 which had been digested with NdeI and BamHI, dephosphorylated and purified by electrophoresis on 1% low melting point gels. The plasmid pTRH44 having a 1.6-kb restriction fragment containing full-length, wild-type ospA gene cloned into pUC9 has been previously described. See Hows, T.R., LaQuier, F.R., & Barbour, A.G., "Organization of genes encoding two outer membrane proteins of the Lyme disease agent Borrelia burgdorferi within a single transcriptional unit," Infec. Immun., 54: 207-212 (1986).

Referring to Figs. 8 and 9, digestion of the amplified DNAs with NdeI/BglII and subsequent ligation into NdeI/BamHI-digested pET9 produced pET9-preOspA and pET9-OspA, which are 5127 and 5082 bps, respectively. 0̸10-S10 represents 0̸10 promoter for bacteriophage T7 RNA polymerase and the ribosome binding and translational start site for T7 gene 10. T0̸ is the transcriptional termination signal for T7 RNA polymerase.

### EXAMPLE 3

For protein production, the plasmids were transferred to the expression strain BL21(DE3)/pLysS, a host strain containing a chromosomal copy of the gene for T7 RNA polymerase under control of the inducible lacUV5 promoter and a pACYC184 based plasmid, pLysS, which specifies low levels of T7 lysozyme, a natural inhibitor of T7 RNA polymerase. For additional information, see Moffatt, B.A., & Studier, F.W., "T7 Lysozyme inhibits transcription by T7 RNA polymerase," cell, 49: 221-227 (1987). In uninduced cells, lysozyme reduces the basal activity of the T7 RNA polymerase and increases the range of target genes that can be stably maintained in the expression host.

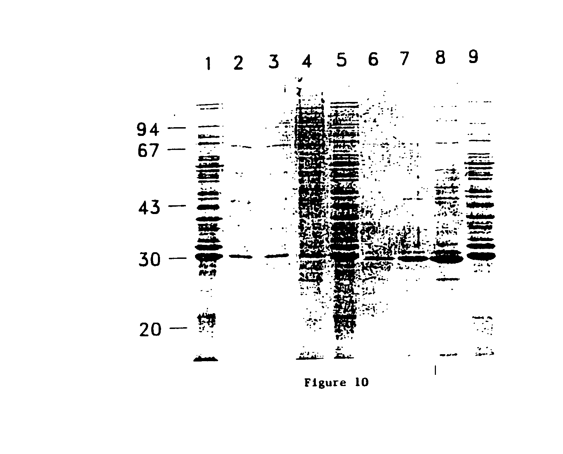

Cultures of BL21(DE3)/pLysS carrying different plasmids were grown to mid-log phase, and a portion from each was induced with IPTG. Upon induction, plasmid pET9-preOspA was found to produce relatively small amounts of inducible protein which from analysis of SDS-polyacrylamide gels was very similar in mobility to wild-type OspA protein present in total extracts of B. buradorferi. Referring to Fig. 10, samples (1.5-*µ*l) were removed for analysis by SDS-12.5% PAGE at the times indicated below. Proteins were visualized by staining with Coomassie blue. Lanes 1, 5 and 9 correspond to whole B. burgdorferi cells (5 x 10⁷ cells) while lanes 2, 3 and 4 correspond to pET9-preOspA induced for 1, 3 or 18 hrs. Lanes 6, 7 and 8 correspond to pET9-OspA induced for 1, 3 or 18 hrs. The position of molecular weight markers (94, 67, 43, 30 and 20) are shown. Molecular masses of proteins are in kilodaltons.

Pulse-chase experiments were performed to demonstrate that synthesis or the preOspA protein caused one to two hours after induction, a result which implied that the protein was toxic to E. coli. In contrast, a much higher and sustained rate of expression was observed when pET9-OspA was induced.

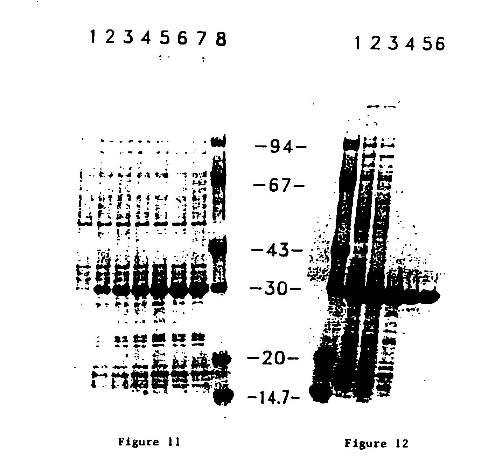

Fig. 11 shows the induction of the recombinant variation of OspA followed by SDS-PAGE. Lane 1 was loaded with whole cells of uninduced BL21(DE3)/pLysS, pET9-OspA. Lanes 2-7 were loaded with whole cells sampled at one hour intervals after induction. Lane 8 contained molecular weight markers. Fig. 12 shows SDS-PAGE of the recombinant version of OspA at different stages of purification as follows: Lane 1, molecular weight markers; Lane 2, crude extract prior to centrifugation; Lane 3, crude extract after centrifugation; Lane 4, Q Sepharose flow through; Lane 5, S Sepharose gradient fraction; and Lane 6, hydroxylapatite fraction. Lanes 2-6 each contain 0.01% of the total protein present in each fraction. Proteins were analyzed on a 10-20% acrylamide gradient gel. Western blot analysis with two monoclonal antibodies, H5332 and H3TS, known to recognize different epitopes within wild-type OspA, was also performed to verify that these bands contained authentic OspA sequences. See Brandt, M.E., Riley, B.S., Radolf, J.D., and Norgard, M.V., "Immunogenic integral membrane proteins of Borrelia burgdorferi are lipoproteins," Infect. Immun., 58: 983-991 (1990). See also Howe, T.R., Mayer, L.W., and Barbour, A.G., "A single recombinant plasmid expressing two major outer surface proteins of the Lyme disease spirochete," Science 227: 645-646 (1985).

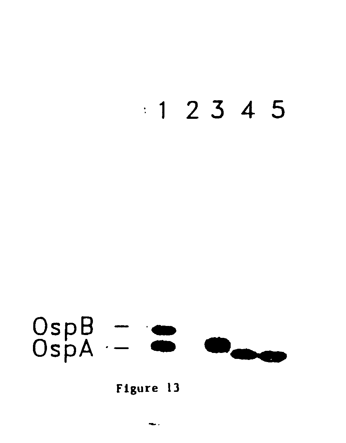

Referring again to Fig. 10, the protein directed to pET9-OspA was noticeably smaller than that produced by pET9-preOspA, even though both proteins were expected to contain approximately the same number of amino-acid residues after processing to remove either the 17 residue long sequence in the case of wild-type OspA or just the initiating methionine from the recombinant variation of OspA. The most likely explanation for the difference was that the presence of covalently attached N-terminal lipid decreased the mobility of the processed pET9-preOspA product. on some gels (see Fig. 13 for example) the preOspA product migrated as two closely spaced bands, which may have represented processing intermediates. This result suggested that the processed and. precursor forms of this protein have similar mobilities on one dimensional SDS-PAGE.

### EXAMPLE 4

In order to determine subcellular localization, a 20mL culture of BL21(DE3)/pLysS, pET9-preOspA was harvested 3½ hours after IPTG induction by centrifugation at 8,000 rpm. The pellet was suspended in a 10 mL mixture containing 20 mM Tris-HCl (pH 8.0), 20 mM NaCl and 2 mM EDTA. The cells were subsequently lysed by freezing and thawing. The lysate was treated with DNase and Mg⁺⁺, then sedimentated for 90 minutes at 33,000 rpm. The pellet fraction was resuspended in a 5 mL mixture containing 0.25 M sucrose, 3.3 mM Tris-HCl (pH 8.0), 1 mM DTT and 1 mM EDTA. The resuspension was re-pelleted by centrifugation for 1 hour at 50,000 rpm. The pellet was resuspended in 1 mL 25% (w/w) sucrose, 5 mM EDTA and 1 mM DTT and subsequently layered onto a discontinuous sucrose gradient. Centrifugation was performed at 30,000 rpm for 16 hours at 4°C. After centrifugation, 0.5 mL fractions were collected and 20 *µ*l samples were analyzed by SDS-PAGE and Western blotting. The outer membrane region of the gradient was determined by its reactivity with antibody to OmpA, a well-characterized E. coli outer membrane component. See Zimmerman, R., and Wickner, W., "Energetics and intermediates of the assembly of protein OmpA into the outer membrane of Escherichia coli," J. Biol. Chem., 258: 3920-3925 (1983).

Almost all the full-length, wild-type OspA resulting from pET9-preOspA was recovered in the lowspeed pellet fraction of the freeze-thaw cell lysate. The pellet was fractionated into inner and outer membranes by centrifugation through discontinuous sucrose gradients. Most of the protein was found in fractions enriched in inner membrane components.

Further studies demonstrated that this recombinantly-derived, wild-type version of OspA could only be extracted under conditions known in the art to selectively solubilize E. coli's inner membrane. Such conditions required the treatment of the protein fraction with a detergent such as Triton X-100 or sodium N-lauryl sarcosinate. For example, see Forst, S., Delgado, J., Ramakrishnan, G., & Inouye, M., "Regulation of ompC and ompF expression in Escherichia coli in the absence of envZ, "J. Bacteriol., 170: 5080-5085 (1988). On the other hand, the product of pET9-OspA, however, is soluble in the absence of any detergent (≥ 50 mg/mL) and significant amounts of this recombinant variation of OspA (≥ 50% of the total cellular protein) can be produced from this plasmid several hours after induction with IPTG (see Fig. 11). when induction of pET9-OspA was continued longer than 6 hours, the cells began to lyse and eventually all the product was found in the culture supernatant.

### EXAMPLE 5

In order to purify the recombinant variation of OspA, a three step procedure was employed. A 500 mL culture of E. coli BL21(DE3)/pLysS containing pET9-OspA was grown in shaking 2-liter flasks at 37°C in tryptone broth supplemented with M9 salts, 0.4% glucose, 25 *µ*g/ml chloramphenicol and 25 *µ*g/mL kanamycin sulfate until the OD 600 reached 0.6, at which point IPTG was added to a final concentration of 0.5 mM. An additional 100 *µ*g/mL kanamycin was added along with the IPTG to prevent overgrowth of the culture by any cells that might have lost the target plasmid. Six hours later, the cells were collected by centrifugation and resuspended in 25-30 mL of 20 mM sodium phosphate buffer (pH 7.7) and stored at -20°C. The crude extract was prepared by thawing the resuspended cells at 4°C, which allows the lysozyme encoded by pLysS to efficiently lyse the cells. This was followed by the addition of MgCl₂ and DNase (final concentrations of 2.5 mM and 5 *µ*g/mL, respectively). After 30 minutes at 4°C, cell debris was removed by centrifugation (15 min, 15,000g). The resulting pellet was extracted with 10 mL of 10 mM sodium phosphate buffer (pH 7.7) containing 10 mM NaCl (buffer A). After recentrifugation, the supernatants were combined to yield approximately 40 mL of crude extract.

The crude extract was applied at room temperature to a prepacked 25-mL bed of Q Sepharose fast flow which had been equilibrated with a buffer A. The column was eluted with 50 mL of buffer A. Essentially all of the target protein was recovered in the flow through buffer.

The fractions containing target protein were dialyzed overnight at 4°C versus 2 x 2-liter changes of 10 mM sodium phosphate buffer (pH 6.0) containing 5 mM NaCl (buffer B), clarified by centrifugation at 10,000g, and then applied at room temperature to a 20 X 1.5 cm column of S Sepharose Fast Flow equilibrated with buffer B. After washing the column with 100 mL of buffer B to remove unbound proteins and contaminants that absorb strongly at 260 nm, the bound target protein was eluted with a linear 300-mL gradient of 0-100 mM NaCl in buffer B, the elution of the target protein occurring at about 35 mM NaCl. Q Sepharose fast flow and S Sepharose fast flow were obtained from Pharmacia, Piscataway, NJ.

The pooled fractions of the target protein resulting from the S Sepharose step were loaded onto a 20 mL bed of Bio-Gel HTP hydroxylapatite previously equilibrated with buffer B. The column was run at room temperature and washed with 50 mL of buffer B. The protein was eluted with a linear 300-mL gradient of 100-400 mM sodium phosphate (pH 6.0). Fractions containing the target protein, which elutes as a broad peak between 150-300 mM sodium phosphate, were pooled and concentrated in an ultrafiltration cell to a final volume of 5 mL. The concentrated protein solution was dialyzed against 10 mM sodium phosphate (pH 6.0), 50 mM NaCl (buffer D) and stored at 4°C. Bio-Gel HTP hydroxyapatite was purchased from Bio-Rad Laboratories, Richmond, CA.

The foregoing method for purification of the recombinant protein product is merely illustrative of a suitable approach for purifying the protein of the present invention. Other suitable techniques known in the art could alternatively be employed for purification. For example, the S Sepharose fraction can be concentrated and applied to a column of Sephacryl S-200 (obtained from Pharmacia, Piscataway, NJ) or other suitable gel filtration matrices.

The resulting yield was 60-70 mg of the recombinant variation of OspA as produced from a 500 mL starting culture. Referring to Fig. 11, this overall yield is approximately 50% as judged from the SDS-PAGE of individual fractions, indicating good hyperexpression of the truncated version of the ospA gene in E. coli.

### EXAMPLE 6

Protein samples of the recombinant variation of OspA were analyzed by polyacrylamide gel electrophoresis under denaturing conditions. For technique, see Studier, F.W., "Analysis of bacteriophage T7 early RNAs and proteins on slab gels," J. Mol. Biol., 79: 237-248 (1973). Gels were fixed and stained with Coomassie blue or the separated proteins were electro-phoretically transferred to nitrocellulose membranes and probed with [¹²⁵I]-labeled protein A for bound immunoglobulin. See Barbour, A.G., "Biology of the Borrelia species," Yale J. Biol. Med., 57, 581-586 (1984). [¹²⁵I]-labeled protein A (5 x 10⁵ cpm/mL) was obtained from DuPont-New England Nuclear.

In some cases, the nitrocellulose membranes were blocked with 3% gelatin in 20 mM Tris-HCl (pH 7.5), 500 mM NaCl (TBS) for a minimum of 1 hour and then washed with TBS containing 0.05% Tween 20 (TTBS) before reaction with antibody. After removal of unbound antibody by several washes in TTBS, reactive proteins were detected by using affinity purified alkaline phosphatase conjugated goat anti-mouse antibody and alkaline phosphatase color development reagents.

The native molecular weight of OspA was determined by chromatography or the purified protein in buffer A containing 200 mM NaCl at a flow rate of 1.5 mL/min on a calibrated 2.5 x 120 cm Sephacryl S-200 column at 4°C. Twenty amino acid residues corresponding to the N-terminal nucleotide sequence were determined using the Edman degradation procedure on a Applied Biosystems 470A Microsequencer. Amino terminal sequencing of the first 20 residues of the recombinant variation of OspA gave a sequence identical with that predicted from the DNA sequence after processing to remove the first methionine residue. The molar extinction coefficient of the protein was calculated from knowledge of its amino-acid composition from the equation E_{M,nat} = (Absnat) (E_{M,Gdn.HCl})/Abs_{Gdn.HCl}). See Gill, S.C., & von Hippel, P.H., "Calculation of protein extinction coefficients from amino acid sequence data," Anal. Biochem. 182: 319-326 (1989). The resulting molar extinction coefficient (E₂₈₀) was 10.59 X 10³ M⁻¹. This value was found to be in excellent agrement (± 5%) with that obtained from analysis of the amino-acid composition of acid hydrolysates, derived from the recombinant variation of OspA, as well as being consistent with the figures regarding the resulting protein yield.

### EXAMPLE 7

The reactivity of the recombinant variation of OspA was tested against human antibodies to the wild-type OspA. Sera from patients with Lyme disease contains antibodies to several B. burgdorferi outer membrane proteins including OspA. Reactive synovial fluid from patients with chronic Lyme disease-related arthritis and OspA-specific antibody H3TS have been previously described. See Barbour, A.G., Burgdorfer, W., Grunwaldt, E., and Steere, A.C., "Antibodies of patients with Lyme disease to components of the Ixodes dammini spirochete," J. Clin. Invest., 72: 504-515 (1983). See also Barbour, A.G., Heiland, R.A., & Howe, T.R., "Heterogeneity of major proteins in Lyme disease borrelia: a molecular analysis of North American and European isolates," J. Infect. Dis., 152: 478-484 (1985).

Referring to Fig. 13, antibodies present in synovial fluid from a patient with Lyme disease-related arthritis reacted strongly in Western blots with the 31-kDa and 34-kDa wild-type OspA and OspB proteins from B. burgdorferi. In addition, both forms of OspA synthesized in E. coli (the recombinant variation or OspA as well as the full-length, wild-type version or OspA synthesized as a control) also reacted strongly.

Referring again to Fig. 13, Lane 1 contained whole cell lysates (2 x 10⁶ cells) of B. burgdorferi (B31) spirochetes while Lanes 2-4 contained induced E. coli cells. Lane 5 contained the purified recombinant variation of OspA (0.5 *µ* g). Samples applied to Lanes 2-5 were derived from cells (6 *µ*L) carrying the pET9 vector plasmid, pET9-preOspA or pET9-OspA, respectively, 3 hours after induction. SDS-12.5% PAGE separated proteins were transferred to nitrocellulose, blocked overnight in 2% bovine serum albumin and then reacted with synovial fluid from a patient with Lyme disease. The blots were washed, incubated with [¹²⁵I]-labeled protein A and then exposed to film for autoradiography. The positions of wild-type B. burgdorferi OspA and OspB are indicated.

While the results show that both plasmids express immunoreactive OspA protein, the protein expressed from pET9-preOspA seemed to react more strongly than an equivalent amount of protein produced from pET9-OspA. Similar results were obtained when Immobilon ™, a hydrophobic polyvinylidene difluoride based membrane, was used as the solid phase for protein blotting. It was postulated that this apparent difference in reactivity might have been caused by poorer transfer to or retention of the recombinant variation of OspA on the nitrocellulose membrane. The foregoing theory was subsequently tested by labeling induced cultures with [³⁵S] methionine (obtained from DuPont-New England Nuclear/specific activity = 1165 Ci/mmol.). Autoradiography was used to follow the relative amounts of wild-type OspA as well as the recombinant variation of OspA present during each step of the Western blot analysis.

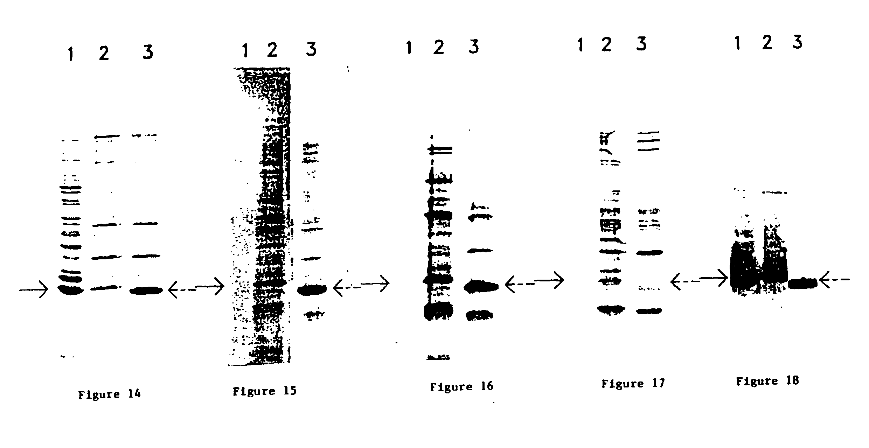

Cultures of BL21(DE3)/pLysS cells carrying pET9-preOspA or pET9-OspA were grown at 37°C to mid-log phase in M9 medium and induced with IPTG. one hour later, each culture was labeled with 20 *µ*Ci/mL [³⁵S]methionine for 5 min and 10 *µ*L portions were analyzed after electrophoresis on 12.5% gels. Referring to Figs. 14 through 18, Lane 1 in each panel contained whole, unlabeled, B. burgdorferi cells (5 x 10⁷ cells). Lanes 2 and 3 contained induced, labeled cells carrying pET9-preOspA or pET9-OspA, respectively. Proteins were visualized by staining with Coomassie blue as shown in Fig. 14 and by autoradiography of the gel as shown in Fig. 15. In Figs. 16 and 17, the proteins are visualized after electrophoretic transfer to nitrocellulose paper. Fig. 16 shows an autoradiogram of the nitrocellulose before further Western analysis. Fig. 17 shows an autoradiogram after probing with antibodies. Fig. 18 shows a photograph of the completed Western blot after treatment with alkaline phosphatase color development reagents. The solid arrows point to the recombinant variation or OspA as well as pET9-preOspA proteins shown in Lanes 1 and 2, respectively. The dashed arrows indicate the position of the pET9-OspA protein product which appears in Lane 3.

Referring now to Figs. 14 through 18, it is apparent that the relative amounts of the wild-type OspA and the recombinant variation of OspA changed during the blotting procedure. The amount of the recombinant variation of OspA which remained after the blot was probed with antibodies and subsequently washed, was considerably reduced relative to the amount initially transferred. In contrast, the wild-type version of OspA was well retained during each step. The results demonstrated that the apparent difference in reactivity of the two forms OspA (the recombinant variation and the wild-type version) was primarily due to a selective loss of the highly soluble, recombinant variation of OspA during Western blot analysis.

### CONCLUSIONS AND SUMMARY OF DATA

While the foregoing method of producing a recombinant version of a protein was directed to outer surface protein A of Borrelia burgdorferi, the method is equally applicable to other Borrelia lipoproteins, provided that the signal sequence for signal peptidase II appears in the gene coding for the lipoprotein in question.

The truncated version of the ospA gene was an excellent overproducer due to its lack of association with the host organism cell membrane. The resulting recombinant variation of OspA accounted for more than 50% of the total cellular protein after a few hours of induction. See examples 4, 5 and 6. In addition, the recombinant variation of OspA is not lipidated and is highly soluble (≥ 50mg/ml) in the absence of detergents. See example 4. Moreover, 60-70 mg of pure protein is available from as little as 0.5 liters of starting culture after a simple purification procedure due to good hyperexpression in the host organism. See examples 5 and 6. Western blots of the recombinant variation of OspA demonstrated that it retained immunoreactive epitopic sites. See examples 3 and 7. This reactivity in conjunction with the highly purified and soluble characteristics of the protein of the present invention make it a good candidate for a diagnostic agent to detect the presence of Lyme disease in clinical isolates as well as a potential immunogen for a vaccine against B. burgdorferi.

Although illustrative embodiments of the present invention have been described herein with reference to the accompanying drawings, it is to be understood that the invention is not limited to those precise embodiments, and that various other changes and modifications may be effected therein by one skilled in the art without deparating from the scope of the invention as hereafter claimed.

## Claims

1. A non-lipidated protein comprising an amino acid sequence which consists of the sequence SEQ ID NO:10 with one or more alanine and/or methionine residues at the N-terminal end of the SEQ ID NO:10 sequence.

2. A protein as claimed in Claim 1, wherein said amino acid sequence is SEQ ID NO:4, SEQ ID NO:6 or SEQ ID NO:8.

3. A protein consisting of an amino acid sequence SEQ ID NO: 10.

4. A nucleotide sequence coding for an amino acid sequence as claimed in any one of Claims 1 to 3.

5. The nucleotide sequence as claimed in Claim 4, coding for an amino acid sequence as claimed in Claim 2, wherein said nucleotide sequence is SEQ ID NO: 3, SEQ ID NO: 5 or SEQ ID NO: 7.

6. The nucleotide sequence as claimed in Claim 4, coding for the amino acid sequence of Claim 3 and wherein said nucleotide sequence is SEQ ID NO: 9.

7. A method for providing a truncated version of a nucleotide sequence coding for the Borrelia burgdorferi lipoprotein OspA, comprising:
providing a DNA template comprising said nucleotide sequence coding for said *Borrelia* lipoprotein, said template containing a potential signal peptidase II signal sequence, wherein said signal sequence has a 5' end and a 3' end, said 3' end terminating in a codon for a cysteine residue; and
synthesizing a set of oligonucleotide primers comprising a first and a second primer, said first primer comprising a region which is sufficiently complementary to a first DNA strand of said DNA template for effectively priming amplification of said first DNA strand in a 5' to 3' direction and a second primer comprising a region which is sufficiently complementary to a second DNA strand of said DNA template for effectively priming amplification of said second DNA strand in a 5' to 3' direction, and using said primers initiates a DNA polymerisation at a nucleotide positioned on said DNA template downstream from said cysteine residue in a 3' direction.

8. The method of Claim 7, wherein said nucleotide is positioned immediately downstream from said cysteine residue in said 3' direction.

9. The method of Claims 7 or 8, wherein said nucleotide sequence of said Borrelia lipoprotein contains a leader sequence having a 5' end and a 3' end.

10. The method of Claim 9, wherein said leader sequence contains or partially contains said signal sequence.

11. The method of Claim 9 or 10, wherein said leader sequence terminates at said 3' end with said signal sequence and said signal sequence is positioned immediately downstream from said 3' end of said leader sequence.

12. A method for providing a recombinant variation of the Borrelia burgdorferi lipoprotein OspA comprising:
providing a DNA template comprising a nucleotide sequence coding for said Borrelia lipoprotein, said template containing a potential signal peptidase II signal sequence, wherein said signal sequence has a 5' end and a 3' end, said 3' end terminating in a codon coding for a cysteine residue;
synthesizing a set of oligonucleotide primers comprising a first and a second primer, said first primer comprising a region which is sufficiently complementary to a first DNA strand of said DNA template for effectively priming amplification of said first DNA strand in a 5' to 3' direction and a second primer comprising a region which is sufficiently complementary to a second DNA strand of said DNA template for effectively priming amplification of said second DNA strand in a 5' to 3' direction, wherein said first primer initiates a DNA polymerization at a nucleotide positioned on said DNA template downstream from said cysteine residue in a 3' direction;
reacting said primers in a polymerase chain reaction thereby providing a set of amplification products;
isolating a truncated version of said nucleotide sequence from said set of amplification products;
cloning said truncated version into a suitable expression vector;
transforming a suitable host organism using said expression vector; and
cultivating said host organism for production of said recombinant variation of said lipoprotein.

13. The method of Claim 12, wherein said expression vector is a plasmid.

14. The method of Claim 13, wherein said expression vector is a pET9 plasmid.

15. The method of Claim 14, wherein said plasmid is pET9-OspA constructed as shown in Figure 9 by the insertion of the truncated OspA nucleotide sequence shown in SEQ ID NO: 10 into plasmid pET9.

16. The method of any one of Claims 12 to 15, wherein said host organism is an *E*.*coli* strain.

17. The method of Claim 16, wherein said *E*. *coli* strain is BL21 (DE3)pLysS.

18. The method of any one of Claims 12 to 17, wherein a T7 bacteriophage expression system is employed.

19. The method of Claim 18, wherein said T7 bacteriophage expression system employs a T7 RNA polymerase.

20. The method of any one of Claims 18 to 19, wherein said *Borrelia* lipoprotein is *a Borrelia burgdorferi* lipoprotein.

21. The method of Claim 20, wherein said *Borrelia burgdorferi* lipoprotein in outer surface protein A (OspA).

22. The method of any one of Claims 7 to 21, wherein said first primer is coded by a nucleotide sequence SEQ ID NO: 11.

23. A transformed strain of *E*. *coli* containing plasmid pET9-OspA as defined in Claim 15, wherein said strain is BL21(DE3)/pLysS, pET0-OspA.

24. An oligonuceotide primer useful for an amplification of a portion of a nucleotide sequence coding for a *Borrelia* lipoprotein, wherein said primer is SEQ ID NO: 11.

## Patentansprüche

1. Nicht-lipidhaltiges Protein, das eine Aminosäuresequenz umfasst, die aus der Sequenz SEQ ID NO:10 mit einem oder mehreren Alanin- und/oder Methioninresten an dem N-terminalen Ende der SEQ ID NO:10-Sequenz besteht.

2. Protein nach Anspruch 1, wobei die Aminosäuresequenz SEQ ID NO:4, SEQ ID NO:6 oder SEQ ID NO:8 ist.

3. Protein, das aus einer Aminosäuresequenz SEQ ID NO:10 besteht.

4. Nukleotidsequenz, die eine Aminosäuresequenz nach einem der Ansprüche 1 bis 3 kodiert.

5. Nukleotidsequenz nach Anspruch 4, die eine Aminosäuresequenz nach Anspruch 2 kodiert, wobei die Nukleotidsequenz SEQ ID NO: 3, SEQ ID NO: 5 oder SEQ ID NO: 7 ist.

6. Nukleotidsequenz nach Anspruch 4, die die Aminosäuresequenz von Anspruch 3 kodiert und wobei die Nukleotidsequenz SEQ ID NO: 9 ist.

7. Verfahren zum Bereitstellen einer verkürzten Version einer Nukleotidsequenz, die das Lipoprotein OspA aus Borrelia burgdorferi kodiert, umfassend:
Bereitstellen einer DNA-Matrize, die die Nukleotidsequenz umfasst, die das *Borrelia*-Lipoprotein kodiert, wobei die Matrize eine potentielle Signalpeptidase II-Signalsequenz enthält, wobei die Signalsequenz ein 5'-Ende und ein 3'-Ende hat, wobei das 3'-Ende mit einem Codon für einen Cysteinrest endet; und
Synthetisieren eines Satzes von Oligonukleotidprimern, der einen ersten und einen zweiten Primer umfasst, wobei der erste Primer eine Region umfasst, die zu einem ersten DNA-Strang der DNA-Matrize ausreichend komplementär ist, um die Amplifizierung des ersten DNA-Strangs in einer 5'→3'-Richtung wirksam zu initiieren bzw. zu primen, und ein zweiter Primer eine Region umfasst, die zu einem zweiten DNA-Strang der DNA-Matrize ausreichend komplementär ist, um die Amplifizierung des zweiten DNA-Strangs in einer 5'→3' -Richtung wirksam zu initiieren bzw. zu primen, und wobei eine Verwendung der Primer eine DNA-Polymerisation an einem Nukleotid, das sich auf der DNA-Matrize stromabwärts von dem Cysteinrest in einer 3'-Richtung befindet, initiiert.

8. Verfahren nach Anspruch 7, wobei das Nukleotid sich unmittelbar stromabwärts von dem Cysteinrest in der 3'-Richtung befindet.

9. Verfahren nach Anspruch 7 oder 8, wobei die Nukleotidsequenz des Borrelia-Lipoproteins eine Leader-Sequenz, die ein 5'-Ende und ein 3'-Ende aufweist, enthält.

10. Verfahren nach Anspruch 9, wobei die Leader-Sequenz die Signalsequenz enthält oder teilweise enthält.

11. Verfahren nach Anspruch 9 oder 10, wobei die Leader-Sequenz an dem 3'-Ende mit der Signalsequenz endet und die Signalsequenz sich unmittelbar stromabwärts von dem 3'-Ende der Leader-Sequenz befindet.

12. Verfahren zum Bereitstellen einer rekombinanten Variante des Lipoproteins OspA aus Borrelia burgdorferi, umfassend:
Bereitstellen einer DNA-Matrize, die eine Nukleotidsequenz umfasst, die das Borrelia-Lipoprotein kodiert, wobei die Matrize eine potentielle Signalpeptidase II-Signalsequenz enthält, wobei die Signalsequenz ein 5'-Ende und ein 3'-Ende hat, wobei das 3'-Ende mit einem Codon, das einen Cysteinrest kodiert, endet;
Synthetisieren eines Satzes von Oligonukleotidprimern, der einen ersten und einen zweiten Primer umfasst, wobei der erste Primer eine Region umfasst, die zu einem ersten DNA-Strang der DNA-Matrize ausreichend komplementär ist, um die Amplifizierung des ersten DNA-Strangs in einer 5 →3'-Richtung wirksam zu initiieren bzw. zu primen, und ein zweiter Primer eine Region umfasst, die zu einem zweiten DNA-Strang der DNA-Matrize ausreichend komplementär ist, um die Amplifizierung des zweiten DNA-Strangs in einer 5'→3' -Richtung wirksam zu initiieren bzw. zu primen, wobei der erste Primer eine DNA-Polymerisation an einem Nukleotid, das sich auf der DNA-Matrize stromabwärts von dem Cysteinrest in einer 3'-Richtung befindet, initiiert;
Reagierenlassen der Primer in einer Polymerasekettenreaktion, wodurch ein Satz von Amplifizierungsprodukten bereitgestellt wird;
Isolieren einer verkürzten Version der Nukleotidsequenz aus dem Satz von Amplifizierungsprodukten;
Klonieren der verkürzten Version in einen geeigneten Ex-pressionsvektor;
Transformieren eines geeigneten Wirtsorganismus unter Verwendung des Expressionsvektors und
Züchten des Wirtsorganismus für eine Produktion der rekombinanten Variante des Lipoproteins.

13. Verfahren nach Anspruch 12, wobei der Expressionsvektor ein Plasmid ist.

14. Verfahren nach Anspruch 13, wobei der Expressionsvektor ein pET9-Plasmid ist.

15. Verfahren nach Anspruch 14, wobei das Plasmid pET9-OspA ist, das, wie in Figur 9 gezeigt, durch die Insertion der in SEQ ID NO: 10 gezeigten verkürzten OspA-Nukleotidsequenz in das Plasmid pET9 konstruiert worden ist.

16. Verfahren nach einem der Ansprüche 12 bis 15, wobei der Wirtsorganismus ein *E*. *coli*-Stamm ist.

17. Verfahren nach Anspruch 16, wobei der *E*. *coli*-Stamm BL21 (DE3)pLysS ist.

18. Verfahren nach einem der Ansprüche 12 bis 17, wobei ein T7-Bakteriophagen-Expressionssystem eingesetzt wird.

19. Verfahren nach Anspruch 18, wobei das T7-Bakteriophagen-Ex-pressionssystem eine T7-RNA-Polymerase einsetzt.

20. Verfahren nach einem der Ansprüche 18 bis 19, wobei das *Borrelia*-Lipoprotein ein *Borrelia burgdorferi*-Lipoprotein ist.

21. Verfahren nach Anspruch 20, wobei das *Borrelia burgdorferi*-Lipoprotein das äußere Oberflächenprotein A (OspA) ist.

22. Verfahren nach einem der Ansprüche 7 bis 21, wobei der erste Primer von einer Nukleotidsequenz SEQ ID NO: 11 kodiert wird.

23. Transformierter *E*. *coli*-Stamm, der das Plasmid pET9-OspA, wie in Anspruch 15 definiert, enthält, wobei der Stamm BL21 (DE3)/pLysS, *p*ET0-OspA ist.

24. Oligonukleotidprimer, der zu einer Amplifizierung eines Abschnitts einer Nukleotidsequenz, die ein *Borrelia*-Lipoprotein kodiert, nützlich ist, wobei der Primer SEQ ID NO: 11 ist.

## Revendications

1. Protéine non lipidique comprenant une séquence d'aminoacides constituée de la séquence SEQ ID N° 10 avec un ou plusieurs résidus d'alanine et/ou de méthionine à l'extrémité N-terminale de la séquence SEQ ID N° 10.

2. Protéine selon la revendication 1, dans laquelle ladite séquence d'aminoacides est SEQ ID N° 4, SEQ ID N° 6 ou SEQ ID N° 8.

3. Protéine constituée d'une séquence d'aminoacides SEQ ID N° 10.

4. Séquence de nucléotides codant pour une séquence d'aminoacides selon l'une quelconque des revendications 1 à 3.

5. Séquence de nucléotides selon la revendication 4, codant pour une séquence d'aminoacides selon la revendication 2, ladite séquence de nucléotides étant SEQ ID N° 3, SEQ ID N° 5 ou SEQ ID N° 7.

6. Séquence de nucléotides selon la revendication 4 codant pour la séquence d'aminoacides de la revendication 3, ladite séquence de nucléotides étant SEQ ID N° 9.

7. Procédé de production d'une version tronquée d'une séquence de nucléotides codant pour la lipoprotéine OspA de *Borrelia burgdorferi*, comprenant:
la fourniture d'une matrice d'ADN comprenant ladite séquence de nucléotides codant pour ladite lipoprotéine de *Borrelia*, ladite matrice contenant une séquence de signal potentielle pour la signal peptidase II, ladite séquence de signal ayant une extrémité 5' et une extrémité 3', ladite extrémité 3' se terminant par un codon codant pour un résidu de cystéine; et
la synthèse d'un ensemble d'amorces oligonucléotidiques comprenant une première et une seconde amorce, ladite première amorce comprenant une région suffisamment complémentaire d'un premier brin d'ADN de ladite matrice d'ADN pour amorcer efficacement l'amplification dudit premier brin d'ADN dans la direction de 5' à 3', et la seconde amorce comprenant une région suffisamment complémentaire d'un second brin d'ADN de ladite matrice d'ADN pour amorcer efficacement l'amplification dudit second brin d'ADN dans la direction de 5' à 3', et l'utilisation desdites amorces amorçant une polymérisation d'ADN au niveau d'un nucléotide situé sur ladite matrice d'ADN en aval dudit résidu de cystéine dans la direction 3'.

8. Procédé selon la revendication 7, dans lequel ledit nucléotide est situé immédiatement en aval dudit résidu de cystéine dans ladite direction 3'.

9. Procédé selon les revendications 7 et 8, dans lequel ladite séquence de nucléotides de ladite lipoprotéine de *Borrelia* contient une séquence de tête ayant une extrémité 5' et une extrémité 3'.

10. Procédé selon la revendication 9, dans lequel ladite séquence de tête contient entièrement ou partiellement ladite séquence de signal.

11. Procédé selon la revendication 9 ou 10, dans lequel ladite séquence de tête se termine à ladite extrémité 3' par ladite séquence de signal et ladite séquence de signal est située immédiatement en aval de ladite extrémité 3' de ladite séquence de tête.

12. Procédé de production d'une variante recombinée de la lipoprotéine OspA de *Borrelia burgdorferi*, comprenant
la fourniture d'une matrice d'ADN comprenant une séquence de nucléotides codant pour ladite lipoprotéine de *Borrelia*, ladite matrice contenant une séquence de signal potentielle pour la signal peptidase II, ladite séquence de signal ayant une extrémité 5' et une extrémité 3', ladite extrémité 3' se terminant par un codon codant pour un résidu de cystéine;
la synthèse d'un ensemble d'amorces oligonucléotidiques comprenant une première et une seconde amorce, ladite première amorce comprenant une région suffisamment complémentaire d'un premier brin d'ADN de ladite matrice d'ADN pour amorcer efficacement l'amplification dudit premier brin d'ADN dans la direction de 5' à 3', et la seconde amorce comprenant une région suffisamment complémentaire d'un second brin d'ADN de ladite matrice d'ADN pour amorcer efficacement l'amplification dudit second brin d'ADN dans la direction de 5' à 3', et ladite première amorce amorçant une polymérisation d'ADN au niveau d'un nucléotide situé sur ladite matrice d'ADN en aval dudit résidu de cystéine dans la direction 3';
la réaction desdites amorces dans une amplification en chaîne par polymérase, ce qui donne un ensemble de produits d'amplification;
l'isolement d'une version tronquée de ladite séquence de nucléotides à partir dudit ensemble de produits d'amplification;
le clonage de ladite version tronquée dans un vecteur d'expression approprié;
la transformation d'un organisme hôte approprié à l'aide dudit vecteur d'expression; et
la culture dudit organisme hôte pour la production de ladite variante recombinée de ladite lipoprotéine.

13. Procédé selon la revendication 12, dans lequel ledit vecteur d'expression est un plasmide.

14. Procédé selon la revendication 13, dans lequel ledit vecteur d'expression est un plasmide pET9.

15. Procédé selon la revendication 14, dans lequel ledit plasmide est pET9-OspA assemblé de la manière indiquée sur la figure 9 par insertion de la séquence de nucléotides d'OspA tronquée indiquée par SEQ ID N° 10 dans le plasmide pET9.

16. Procédé selon l'une quelconque des revendications 12 à 15, dans lequel ledit organisme hôte est une souche d'*E*. *coli*.

17. Procédé selon la revendication 16, dans lequel ladite souche d'*E*. *coli* est la souche BL21 (DE3)pLysS.

18. Procédé selon l'une quelconque des revendications 12 à 17, dans lequel on utilise un système d'expression du bactériophage T7.

19. Procédé selon la revendication 18, dans lequel ledit système d'expression du bactériophage T7 utilise une ARN polymérase de T7.

20. Procédé selon l'une quelconque des revendications 18 à 19, dans lequel ladite lipoprotéine de *Borrelia* est une lipoprotéine de *Borrelia burgdorferi*.

21. Procédé selon la revendication 20, dans lequel ladite lipoprotéine de *Borrelia burgdorferi* est la protéine A de la surface externe (OspA).

22. Procédé selon l'une quelconque des revendications 7 à 21, dans lequel ladite première amorce est codée par la séquence de nucléotides SEQ ID N° 11.

23. Souche transformée de *E*. *coli* contenant le plasmide pTE9-OspA tel que défini dans la revendication 15, ladite souche étant BL21 (DE3)/pLysS, pET9-OspA.

24. Amorce oligonucléotidique utile pour l'amplification d'une portion d'une séquence de nucléotides codant pour une lipoprotéine de *Borrelia burgdorferi*, ladite amorce étant SEQ ID N° 11.


### SEQUENCE LISTING

(1) GENERAL INFORMATION:
   (i) APPLICANT: Dunn, John J.
   (ii) TITLE OF INVENTION: Cloning and Expression of of Borrelia Lipoproteins
   (iii) NUMBER OF SEOUENCES: 15
   (iv) CORRESPONDENCE ADDRESS:
      (A) ADDRESSEE: Brookhaven National Laboratory Associated Universities, Inc.
      (B) STREET:
      (C) CITY: Upton
      (D) STATE: New York
      (E) COUNTRY: United States of America
      (F) ZIP: 11973
   (v) COMPUTER READABLE FORM:
      (A) MEDIUM TYPE: Diskette - 5.25 inch, 360 Kb
      (B) COMPUTER: IBM XT compatible
      (C) OPERATING SYSTEM: MS DOS
      (D) SOFTWARE: WORD PERFECT 4.2
(2) INFORMATION FOR SEQ ID NO: 1 :
   (i) SEQUENCE CHARACTERISTICS:
      (A) LENGTH: 822 bases
      (B) TYPE: Nucleic acid
      (C) STRANDEDNESS: Double stranded
      (D) TOPOLOGY: Linear
   (xi) SEQUENCE DESCRIPTION: SEQ ID NO: 1 :
   ATG AAA AAA TAT TTA TTG GGA ATA GGT CTA ATA TTA GCC TTA ATA GCA     48
   Met Lys Lys Tyr Leu Leu Gly Ile Gly Leu Ile Leu Ala Leu Ile Ala
    1               5                  10                  15
   TGT AAG CAA AAT GTT AGC AGC CTT GAC GAG AAA AAC AGC GTT TCA GTA     96
   Cys Lys Gln Asn Val Ser Ser Leu Asp Glu Lys Asn Ser Val Ser Val
               20                  25                  30
   GAT TTG CCT GGT GAA ATG AAA GTT CTT GTA AGC AAA GAA AAA AAC AAA    144
   Asp Leu Pro Gly Glu Met Lys Val Leu Val Ser Lys Glu Lys Asn Lys
           35                  40                  45
   GAC GGC AAG TAC GAT CTA ATT GCA ACA GTA GAC AAG CTT GAG CTT AAA    192
   Asp Gly Lys Tyr Asp Leu Ile Ala Thr Val Asp Lys Leu Glu Leu Lys
       50                  55                  60
   GGA ACT TCT GAT AAA AAC AAT GGA TCT GGA GTA CTT GAA GGC GTA AAA    240
   Gly Thr Ser Asp Lys Asn Asn Gly Ser Gly Val Leu Glu Gly Val Lys
   65                  70                  75                  80
   GCT GAC AAA AGT AAA GTA AAA TTA ACA ATT TCT GAC GAT CTA GGT CAA    288
   Ala Asp Lys Ser Lys Val Lys Leu Thr Ile Ser Asp Asp Leu Gly Gln
                   85                  90                  95
   ACC ACA CTT GAA GTT TTC AAA GAA GAT GGC AAA ACA CTA GTA TCA AAA    336
   Thr Thr Leu Glu Val Phe Lys Glu Asp Gly Lys Thr Leu Val Ser Lys
               100                 105                 110
   AAA GTA ACT TCC AAA GAC AAG TCA TCA ACA GAA GAA AAA TTC AAT GAA    384
   Lys Val Thr Ser Lys Asp Lys Ser Ser Thr Glu Glu Lys Phe Asn Glu
           115                 120                 125
   AAA GGT GAA GTA TCT GAA AAA ATA ATA ACA AGA GCA GAC GGA ACC AGA    432
   Lys Gly Glu Val Ser Glu Lys Ile Ile Thr Arg Ala Asp Gly Thr Arg
       130                 135                 140
   CTT GAA TAC ACA GGA ATT AAA AGC GAT GGA TCT GGA AAA GCT AAA GAG    480
   Leu Glu Tyr Thr Gly Ile Lys Ser Asp Gly Ser Gly Lys Ala Lys Glu
   145                 150                 155                 160
   GTT TTA AAA GGC TAT GTT CTT GAA GGA ACT CTA ACT GCT GAA AAA ACA    528
   Val Leu Lys Gly Tyr Val Leu Glu Gly Thr Leu Thr Ala Glu Lys Thr
                   165                 170                 175
   ACA TTG GTG GTT AAA GAA GGA ACT GTT ACT TTA AGC AAA AAT ATT TCA    576
   Thr Leu Val Val Lys Glu Gly Thr Val Thr Leu Ser Lys Asn Ile Ser
               180                 185                 190
   AAA TCT GGG GAA GTT TCA GTT GAA CTT AAT GAC ACT GAC AGT AGT GCT    624
   Lys Ser Gly Glu Val Ser Val Glu Leu Asn Asp Thr Asp Ser Ser Ala
           195                 200                 205
   GCT ACT AAA AAA ACT GCA GCT TGG AAT TCA GGC ACT TCA ACT TTA ACA    672
   Ala Thr Lys Lys Thr Ala Ala Trp Asn Ser Gly Thr Ser Thr Leu Thr
       210                 215                 220
   ATT ACT GTA AAC AGT AAA AAA ACT AAA GAC CTT GTG TTT ACA AAA GAA    720
   Ile Thr Val Asn Ser Lys Lys Thr Lys Asp Leu Val Phe Thr Lys Glu
   225                 230                 235                 240
   AAC ACA ATT ACA GTA CAA CAA TAC GAC TCA AAT GGC ACC AAA TTA GAG    768
   Asn Thr Ile Thr Val Gln Gln Tyr Asp Ser Asn Gly Thr Lys Leu Glu
                   245                 250                 255
   GGG TCA GCA GTT GAA ATT ACA AAA CTT GAT GAA ATT AAA AAC GCT TTA    816
   Gly Ser Ala Val Glu Ile Thr Lys Leu Asp Glu Ile Lys Asn Ala Leu
               260                 265                 270
   AAA TAA                                                            822
   Lys
(2) INFORMATION FOR SEQ ID NO: 2 :
   (i) SEQUENCE CHARACTERISTICS:
      (A) LENGTH: 273 Amino acids
      (B) TYPE: Amino acid
      (D) TOPOLOGY: Linear
   (xi) SEQUENCE DESCRIPTION: SEQ ID NO: 2 :
(2) INFORMATION FOR SEQ ID NO: 3 :
   (i) SEQUENCE CHARACTERISTICS:
      (A) LENGTH: 777 bases
      (B) TYPE: Nucleic acid
      (C) STRANDEDNESS: Double stranded
      (D) TOPOLOGY: Linear
   (xi) SEQUENCE DESCRIPTION: SEQ ID NO: 3 :
(2) INFORMATION FOR SEQ ID NO: 4 :
   (i) SEQUENCE CHARACTERISTICS:
      (A) LENGTH: 258 Amino acids
      (B) TYPE: Amino acid
      (D) TOPOLOGY: Linear
   (xi) SEQUENCE DESCRIPTION: SEQ ID NO: 4 :
   Met Ala Lys Gln Asn Val Ser Ser Leu Asp Glu Lys Asn Ser Val Ser
   1 5 10 15
   Val Asp Leu Pro Gly Glu Met Lys Val Leu Val Ser Lys Glu Lys Asn
   20 25 30
   Lys Asp Gly Lys Tyr Asp Leu Ile Ala Thr Val Asp Lys Leu Glu Leu
   35 40 45
   Lys Gly Thr Ser Asp Lys Asn Asn Gly Ser Gly Val Leu Glu Gly Val
   50 55 60
   Lys Ala Asp Lys Ser Lys Val Lys Leu Thr Ile Ser Asp Asp Leu Gly
   65 70 75 80
   Gln Thr Thr Leu Glu Val Phe Lys Glu Asp Gly Lys Thr Leu Val Ser
   85 90 95
   Lys Lys Val Thr Ser Lys Asp Lys Ser Ser Thr Glu Glu Lys Phe Asn
   100 105 110
   Glu Lys Gly Glu Val Ser Glu Lys Ile Ile Thr Arg Ala Asp Gly Thr
   115 120 125
   Arg Leu Glu Tyr Thr Gly Ile Lys Ser Asp Gly Ser Gly Lys Ala Lys
   130 135 140
   Glu Val Leu Lys Gly Tyr Val Leu Glu Gly Thr Leu Thr Ala Glu Lys
   145 150 155 160
   Thr Thr Leu Val Val Lys Glu Gly Thr Val Thr Leu Ser Lys Asn Ile
   165 170 175
   Ser Lys Ser Gly Glu Val Ser Val Glu Leu Asn Asp Thr Asp Ser Ser
   180 185 190
   Ala Ala Thr Lys Lys Thr Ala Ala Trp Asn Ser Gly Thr Ser Thr Leu
   195 200 205
   Thr Ile Thr Val Asn Ser Lys Lys Thr Lys Asp Leu Val Phe Thr Lys
   210 215 220
   Glu Asn Thr Ile Thr Val Gln Gln Tyr Asp Ser Asn Gly Thr Lys Leu
   225 230 235 240
   Glu Gly Ser Ala Val Glu Ile Thr Lys Leu Asp Glu Ile Lys Asn Ala
   245 250 255
   Leu Lys
(2) INFORMATION FOR SEQ ID NO: 5 :
   (i) SEQUENCE CHARACTERISTICS:
      (A) LENGTH: 774 bases
      (B) TYPE: Nucleic acid
      (C) STRANDEDNESS: Double stranded
      (D) TOPOLOGY: Linear
   (xi) SEQUENCE DESCRIPTION: SEQ ID NO: 5 :
   GCT AAG CAA AAT GTT AGC AGC CTT GAC GAG AAA AAC AGC GTT TCA GTA    48
   Ala Lys Gln Asn Val Ser Ser Leu Asp Glu Lys Asn Ser Val Ser Val
    1               5                  10                  15
   GAT TTG CCT GGT GAA ATG AAA GTT CTT GTA AGC AAA GAA AAA AAC AAA    96
   Asp Leu Pro Gly Glu Met Lys Val Leu Val Ser Lys Glu Lys Asn Lys
               20                  25                  30
   GAC GGC AAG TAC GAT CTA ATT GCA ACA GTA GAC AAG CTT GAG CTT AAA   144
   Asp Gly Lys Tyr Asp Leu Ile Ala Thr Val Asp Lys Leu Glu Leu Lys
           35                  40                  45
   GGA ACT TCT GAT AAA AAC AAT GGA TCT GGA GTA CTT GAA GGC GTA AAA   192
   Gly Thr Ser Asp Lys Asn Asn Gly Ser Gly Val Leu Glu Gly Val Lys
       50                  55                  60
   GCT GAC AAA AGT AAA GTA AAA TTA ACA ATT TCT GAC GAT CTA GGT CAA   240
   Ala Asp Lys Ser Lys Val Lys Leu Thr Ile Ser Asp Asp Leu Gly Gln
   65                  70                  75                  80
   ACC ACA CTT GAA GTT TTC AAA GAA GAT GGC AAA ACA CTA GTA TCA AAA   288
   Thr Thr Leu Glu Val Phe Lys Glu Asp Gly Lys Thr Leu Val Ser Lys
                   85                  90                  95
   AAA GTA ACT TCC AAA GAC AAG TCA TCA ACA GAA GAA AAA TTC AAT GAA   336
   Lys Val Thr Ser Lys Asp Lys Ser Ser Thr Glu Glu Lys Phe Asn Glu
               100                 105                 110
   AAA GGT GAA GTA TCT GAA AAA ATA ATA ACA AGA GCA GAC GGA ACC AGA   384
   Lys Gly Glu Val Ser Glu Lys Ile Ile Thr Arg Ala Asp Gly Thr Arg
           115                 120                 125
   CTT GAA TAC ACA GGA ATT AAA AGC GAT GGA TCT GGA AAA GCT AAA GAG   432
   Leu Glu Tyr Thr Gly Ile Lys Ser Asp Gly Ser Gly Lys Ala Lys Glu
       130                 135                 140
   GTT TTA AAA GGC TAT GTT CTT GAA GGA ACT CTA ACT GCT GAA AAA ACA   480
   Val Leu Lys Gly Tyr Val Leu Glu Gly Thr Leu Thr Ala Glu Lys Thr
   145                 150                 155                 160
   ACA TTG GTG GTT AAA GAA GGA ACT GTT ACT TTA AGC AAA AAT ATT TCA   528
   Thr Leu Val Val Lys Glu Gly Thr Val Thr Leu Ser Lys Asn Ile Ser
                   165                 170                 175
   AAA TCT GGG GAA GTT TCA GTT GAA CTT AAT GAC ACT GAC AGT AGT GCT   576
   Lys Ser Gly Glu Val Ser Val Glu Leu Asn Asp Thr Asp Ser Ser Ala
               180                 185                 190
   GCT ACT AAA AAA ACT GCA GCT TGG AAT TCA GGC ACT TCA ACT TTA ACA   624
   Ala Thr Lys Lys Thr Ala Ala Trp Asn Ser Gly Thr Ser Thr Leu Thr
           195                 200                 205
   ATT ACT GTA AAC AGT AAA AAA ACT AAA GAC CTT GTG TTT ACA AAA GAA      672
   Ile Thr Val Asn Ser Lys Lys Thr Lys Asp Leu Val Phe Thr Lys Glu
       210                 215                 220
   AAC ACA ATT ACA GTA CAA CAA TAC GAC TCA AAT GGC ACC AAA TTA GAG      720
   Asn Thr Ile Thr Val Gln Gln Tyr Asp Ser Asn Gly Thr Lys Leu Glu
   225                 230                 235                 240
   GGG TCA GCA GTT GAA ATT ACA AAA CTT GAT GAA ATT AAA AAC GCT TTA      768
   Gly Ser Ala Val Glu Ile Thr Lys Leu Asp Glu Ile Lys Asn Ala Leu
                   245                 250                 255
   AAA TAA                                                              774
   Lys
(2) INFORMATION FOR SEQ ID NO: 6 :
   (i) SEQUENCE CHARACTERISTICS:
      (A) LENGTH: 257 Amino acids
      (B) TYPE: Amino acid
      (D) TOPOLOGY: Linear
   (xi) SEQUENCE DESCRIPTION: SEQ ID NO: 6 :
(2) INFORMATION FOR SEQ ID NO: 7 :
   (i) SEQUENCE CHARACTERISTICS:
      (A) LENGTH: 774 bases
      (B) TYPE: Nucleic acid
      (C) STRANDEDNESS: Double stranded
      (D) TOPOLOGY: Linear
   (xi) SEQUENCE DESCRIPTION: SEQ ID NO: 7 :
   ATG AAG CAA AAT GTT AGC AGC CTT GAC GAG AAA AAC AGC GTT TCA GTA     48
   Met Lys Gln Asn Val Ser Ser Leu Asp Glu Lys Asn Ser Val Ser Val
     1               5                  10                  15
   GAT TTG CCT GGT GAA ATG AAA GTT CTT GTA AGC AAA GAA AAA AAC AAA     96
   Asp Leu Pro Gly Glu Met Lys Val Leu Val Ser Lys Glu Lys Asn Lys
                20                  25                  30
   GAC GGC AAG TAC GAT CTA ATT GCA ACA GTA GAC AAG CTT GAG CTT AAA    144
   Asp Gly Lys Tyr Asp Leu Ile Ala Thr Val Asp Lys Leu Glu Leu Lys
            35                  40                  45
   GGA ACT TCT GAT AAA AAC AAT GGA TCT GGA GTA CTT GAA GGC GTA AAA    192
   Gly Thr Ser Asp Lys Asn Asn Gly Ser Gly Val Leu Glu Gly Val Lys
        50                  55                  60
   GCT GAC AAA AGT AAA GTA AAA TTA ACA ATT TCT GAC GAT CTA GGT CAA    240
   Ala Asp Lys Ser Lys Val Lys Leu Thr Ile Ser Asp Asp Leu Gly Gln
    65                  70                  75                  80
   ACC ACA CTT GAA GTT TTC AAA GAA GAT GGC AAA ACA CTA GTA TCA AAA    288
   Thr Thr Leu Glu Val Phe Lys Glu Asp Gly Lys Thr Leu Val Ser Lys
                    85                  90                  95
   AAA GTA ACT TCC AAA GAC AAG TCA TCA ACA GAA GAA AAA TTC AAT GAA    336
   Lys Val Thr Ser Lys Asp Lys Ser Ser Thr Glu Glu Lys Phe Asn Glu
               100                 105                 110
   AAA GGT GAA GTA TCT GAA AAA ATA ATA ACA AGA GCA GAC GGA ACC AGA    384
   Lys Gly Glu Val Ser Glu Lys Ile Ile Thr Arg Ala Asp Gly Thr Arg
           115                 120                 125
   CTT GAA TAC ACA GGA ATT AAA AGC GAT GGA TCT GGA AAA GCT AAA GAG    432
   Leu Glu Tyr Thr Gly Ile Lys Ser Asp Gly Ser Gly Lys Ala Lys Glu
       130                 135                 140
   GTT TTA AAA GGC TAT GTT CTT GAA GGA ACT CTA ACT GCT GAA AAA ACA     480
   Val Leu Lys Gly Tyr Val Leu Glu Gly Thr Leu Thr Ala Glu Lys Thr
   145                 150                 155                 160
   ACA TTG GTG GTT AAA GAA GGA ACT GTT ACT TTA AGC AAA AAT ATT TCA     528
   Thr Leu Val Val Lys Glu Gly Thr Val Thr Leu Ser Lys Asn Ile Ser
                   165                 170                 175
   AAA TCT GGG GAA GTT TCA GTT GAA CTT AAT GAC ACT GAC AGT AGT GCT     576
   Lys Ser Gly Glu Val Ser Val Glu Leu Asn Asp Thr Asp Ser Ser Ala
               180                 185                 190
   GCT ACT AAA AAA ACT GCA GCT TGG AAT TCA GGC ACT TCA ACT TTA ACA     624
   Ala Thr Lys Lys Thr Ala Ala Trp Asn Ser Gly Thr Ser Thr Leu Thr
           195                 200                 205
   ATT ACT GTA AAC AGT AAA AAA ACT AAA GAC CTT GTG TTT ACA AAA GAA     672
   Ile Thr Val Asn Ser Lys Lys Thr Lys Asp Leu Val Phe Thr Lys Glu
       210                 215                 220
   AAC ACA ATT ACA GTA CAA CAA TAC GAC TCA AAT GGC ACC AAA TTA GAG     720
   Asn Thr Ile Thr Val Gln Gln Tyr Asp Ser Asn Gly Thr Lys Leu Glu
   225                 230                 235                 240
   GGG TCA GCA GTT GAA ATT ACA AAA CTT GAT GAA ATT AAA AAC GCT TTA     768
   Gly Ser Ala Val Glu Ile Thr Lys Leu Asp Glu Ile Lys Asn Ala Leu
                   245                 250                 255
   AAA TAA                                                             774
   Lys
(2) INFORMATION FOR SEQ ID NO: 8 :
   (i) SEQUENCE CHARACTERISTICS:
      (A) LENGTH: 257 Amino acids
      (B) TYPE: Amino acid
      (D) TOPOLOGY: Linear
   (xi) SEQUENCE DESCRIPTION: SEQ ID NO: 8 :
   Met Lys Gln Asn Val Ser Ser Leu Asp Glu Lys Asn Ser Val Ser Val
    1               5                  10                  15
   Asp Leu Pro Gly Glu Met Lys Val Leu Val Ser Lys Glu Lys Asn Lys
               20                  25                  30
   Asp Gly Lys Tyr Asp Leu Ile Ala Thr Val Asp Lys Leu Glu Leu Lys
           35                  40                  45
   Gly Thr Ser Asp Lys Asn Asn Gly Ser Gly Val Leu Glu Gly Val Lys
       50                  55                  60
   Ala Asp Lys Ser Lys Val Lys Leu Thr Ile Ser Asp Asp Leu Gly Gln
   65                  70                  75                  80
   Thr Thr Leu Glu Val Phe Lys Glu Asp Gly Lys Thr Leu Val Ser Lys
                   85                  90                  95
   Lys Val Thr Ser Lys Asp Lys Ser Ser Thr Glu Glu Lys Phe Asn Glu
               100                 105                 110
   Lys Gly Glu Val Ser Glu Lys Ile Ile Thr Arg Ala Asp Gly Thr Arg
           115                 120                 125
   Leu Glu Tyr Thr Gly Ile Lys Ser Asp Gly Ser Gly Lys Ala Lys Glu
       130                 135                 140
   Val Leu Lys Gly Tyr Val Leu Glu Gly Thr Leu Thr Ala Glu Lys Thr
   145                 150                 155                 160
   Thr Leu Val Val Lys Glu Gly Thr Val Thr Leu Ser Lys Asn Ile Ser
                   165                 170                 175
   Lys Ser Gly Glu Val Ser Val Glu Leu Asn Asp Thr Asp Ser Ser Ala
               180                 185                 190
   Ala Thr Lys Lys Thr Ala Ala Trp Asn Ser Gly Thr Ser Thr Leu Thr
           195                 200                 205
   Ile Thr Val Asn Ser Lys Lys Thr Lys Asp Leu Val Phe Thr Lys Glu
       210                 215                 220
   Asn Thr Ile Thr Val Gln Gln Tyr Asp Ser Asn Gly Thr Lys Leu Glu
   225                 230                 235                 240
   Gly Ser Ala Val Glu Ile Thr Lys Leu Asp Glu Ile Lys Asn Ala Leu
                   245                 250                 255
   Lys
(2) INFORMATION FOR SEQ ID NO: 9 :
   (i) SEQUENCE CHARACTERISTICS:
      (A) LENGTH: 771 bases
      (B) TYPE: Nucleic acid
      (C) STRANDEDNESS: Double stranded
      (D) TOPOLOGY: Linear
   (xi) SEQUENCE DESCRIPTION: SEQ ID NO: 9 :
   AAG CAA AAT GTT AGC AGC CTT GAC GAG AAA AAC AGC GTT TCA GTA GAT     48
   Lys Gln Asn Val Ser Ser Leu Asp Glu Lys Asn Ser Val Ser Val Asp
     1               5                  10                  15
   TTG CCT GGT GAA ATG AAA GTT CTT GTA AGC AAA GAA AAA AAC AAA GAC     96
   Leu Pro Gly Glu Met Lys Val Leu Val Ser Lys Glu Lys Asn Lys Asp
                20                  25                  30
   GGC AAG TAC GAT CTA ATT GCA ACA GTA GAC AAG CTT GAG CTT AAA GGA    144
   Gly Lys Tyr Asp Leu Ile Ala Thr Val Asp Lys Leu Glu Leu Lys Gly
            35                  40                  45
   ACT TCT GAT AAA AAC AAT GGA TCT GGA GTA CTT GAA GGC GTA AAA GCT    192
   Thr Ser Asp Lys Asn Asn Gly Ser Gly Val Leu Glu Gly Val Lys Ala
        50                  55                  60
   GAC AAA AGT AAA GTA AAA TTA ACA ATT TCT GAC GAT CTA GGT CAA ACC   240
   Asp Lys Ser Lys Val Lys Leu Thr Ile Ser Asp Asp Leu Gly Gln Thr
   65                  70                  75                  80
   ACA CTT GAA GTT TTC AAA GAA GAT GGC AAA ACA CTA GTA TCA AAA AAA      288
   Thr Leu Glu Val Phe Lys Glu Asp Gly Lys Thr Leu Val Ser Lys Lys
                    85                  90                  95
   GTA ACT TCC AAA GAC AAG TCA TCA ACA GAA GAA AAA TTC AAT GAA AAA   336
   Val Thr Ser Lys Asp Lys Ser Ser Thr Glu Glu Lys Phe Asn Glu Lys
               100                     105                     110
   GGT GAA GTA TCT GAA AAA ATA ATA ACA AGA GCA GAC GGA ACC AGA CTT   384
   Gly Glu Val Ser Glu Lys Ile Ile Thr Arg Ala Asp Gly Thr Arg Leu
           115                     120                     125
   GAA TAC ACA GGA ATT AAA AGC GAT GGA TCT GGA AAA GCT AAA GAG GTT    432
   Glu Tyr Thr Gly Ile Lys Ser Asp Gly Ser Gly Lys Ala Lys Glu Val
       130                 135                 140
   TTA AAA GGC TAT GTT CTT GAA GGA ACT CTA ACT GCT GAA AAA ACA ACA    480
   Leu Lys Gly Tyr Val Leu Glu Gly Thr Leu Thr Ala Glu Lys Thr Thr
   145                 150                 155                 160
   TTG GTG GTT AAA GAA GGA ACT GTT ACT TTA AGC AAA AAT ATT TCA AAA    528
   Leu Val Val Lys Glu Gly Thr Val Thr Leu Ser Lys Asn Ile Ser Lys
                   165                 170                 175
   TCT GGG GAA GTT TCA GTT GAA CTT AAT GAC ACT GAC AGT AGT GCT GCT    576
   Ser Gly Glu Val Ser Val Glu Leu Asn Asp Thr Asp Ser Ser Ala Ala
               180                 185                 190
   ACT AAA AAA ACT GCA GCT TGG AAT TCA GGC ACT TCA ACT TTA ACA ATT    624
   Thr Lys Lys Thr Ala Ala Trp Asn Ser Gly Thr Ser Thr Leu Thr Ile
           195                 200                 205
   ACT GTA AAC AGT AAA AAA ACT AAA GAC CTT GTG TTT ACA AAA GAA AAC    672
   Thr Val Asn Ser Lys Lys Thr Lys Asp Leu Val Phe Thr Lys Glu Asn
       210                 215                 220
   ACA ATT ACA GTA CAA CAA TAC GAC TCA AAT GGC ACC AAA TTA GAG GGG    720
   Thr Ile Thr Val Gln Gln Tyr Asp Ser Asn Gly Thr Lys Leu Glu Gly
   225                 230                 235                 240
   TCA GCA GTT GAA ATT ACA AAA CTT GAT GAA ATT AAA AAC GCT TTA AAA    768
   Ser Ala Val Glu Ile Thr Lys Leu Asp Glu Ile Lys Asn Ala Leu Lys
                   245                 250                 255
   TAA                                                                771
(2) INFORMATION FOR SEQ ID NO: 10
   (i) SEQUENCE CHARACTERISTICS:
      (A) LENGTH: 256 Amino acids
      (B) TYPE: Amino acid
      (D) TOPOLOGY: Linear
   (xi) SEQUENCE DESCRIPTION: SEQ ID NO: 10
(2) INFORMATION FOR SEQ ID NO: 11
   (i) SEQUENCE CHARACTERISTICS:
      (A) LENGTH: 32 bases
      (B) TYPE: Nucleic acid
      (C) STRANDEDNESS: Single stranded
      (D) TOPOLOGY: Linear
   (xi) SEQUENCE DESCRIPTION: SEQ ID NO: 11
   CCGGGATCCA T ATG GCT AAG CAA AAT GTT AGC        32
                Met Ala Lys Gln Asn Val Ser
                 1               5
(2) INFORMATION FOR SEQ ID NO: 12 :
   (i) SEQUENCE CHARACTERISTICS:
      (A) LENGTH: 28 bases
      (B) TYPE: Nucleic acid
      (C) STRANDEDNESS: Single stranded
      (D) TOPOLOGY: Linear
   (xi) SEQUENCE DESCRIPTION: SEQ ID NO: 12 :
   GATATCTAGA TCTTTATTTT AAAGCGTT        28
(2) INFORMATION FOR SEQ ID NO: 13
   (i) SEQUENCE CHARACTERISTICS:
      (A) LENGTH: 31 bases
      (B) TYPE: Nucleic acid
      (C) STRANDEDNESS: Single stranded
      (D) TOPOLOGY: Linear
   (xi) SEQUENCE DESCRIPTION: SEQ ID NO: 13 :
   CCGGATCCAT ATG AAA AAA TAT TTA TTG GGA        31
              Met Lys Lys Tyr Leu Leu Gly
               1               5
(2) INFORMATION FOR SEQ ID NO: 14
   (i) SEQUENCE CHARACTERISTICS:
      (A) LENGTH: 12 bases
      (B) TYPE: Nucleic acid
      (C) STRANDEDNESS: Double stranded
      (D) TOPOLOGY: Linear
   (xi) SEQUENCE DESCRIPTION: SEQ ID NO: 14
   TTA ATA GCA TGT 12
   Leu Ile Ala Cys
   1
(2) INFORMATION FOR SEQ ID NO: 15 :
   (i) SEQUENCE CHARACTERISTICS:
      (A) LENGTH: 4 Amino acids
      (B) TYPE: Amino acid
      (D) TOPOLOGY: Linear
   (xi) SEQUENCE DESCRIPTION: SEQ ID NO: 15
   Leu Ile Ala Cys
   1